# EUROPEAN PATENT SPECIFICATION

(11) **EP 1 627 055 B1**
(45) Date of publication and mention of the grant of the patent: **27.03.2013**
(21) Application number: 04733060.0
(22) Date of filing: 14.05.2004
(51) Int. Cl.: C12N 5/0784, A61K 35/14, A61P 31/00

(54) **NOVEL USES OF PPAR MODULATORS AND PROFESSIONAL APCS MANIPULATED BY THE SAME**
NEUARTIGE VERWENDUNGEN VON PPAR-MODULATOREN UND DAMIT MANIPULIERTE PROFESSIONELLE APCS
NOUVEAUX EMPLOIS DES MODULATEURS DU RECEPTEUR PPAR ET CELLULES PRESENTATRICES D'ANTIGENES PROFESSIONNELLES MANIPULEES PAR LESDITS MODULATEURS

(30) Priority: 14.05.2003 HU 0301358
(43) Date of publication of application: 22.02.2006
(73) Proprietor: University of Debrecen, 4012 Debrecen (HU)
(72) Inventor: NAGY, László, 4028 Debrecen (HU); SZATMÁRI, István, 4032 Debrecen (HU); RAJNAVÖLGYI, Éva, 1025 Budapest (HU); GOGOLÁK, Péter, 1045 Budapest (HU); RÉTHI, Bence, 1131 Budapest (HU)
(74) Representative: Svingor, Adam
(86) International application number: PCT/IB2004/050707
(87) International publication number: WO 2004/101776

(56) References cited:
- WO-A-00/62787
- WO-A-98/29113
- WO-A-02/076177
- MACH NICOLAS ET AL: "Differences in dendritic cells stimulated in vivo by tumors engineered to secrete granulocyte-macrophage colony-stimulating factor or Flt3-ligand" CANCER RESEARCH, AMERICAN ASSOCIATION FOR CANCER RESEARCH, BALTIMORE, MD, US, vol. 60, no. 12, 15 June 2000 (2000-06-15), pages 3239-3246, XP002258981 ISSN: 0008-5472
- NENCIONI ALESSIO ET AL: "Dendritic cell immunogenicity is regulated by peroxisome proliferator-activated receptor gamma." JOURNAL OF IMMUNOLOGY (BALTIMORE, MD. : 1950) 1 AUG 2002, vol. 169, no. 3, 1 August 2002 (2002-08-01), pages 1228-1235, XP002307075 ISSN: 0022-1767 cited in the application
- HONG S ET AL: "LIPID ANTIGEN PRESENTATION IN THE IMMUNE SYSTEM: LESSONS LEARNED FROM CD1D KNOCKOUT MICE" IMMUNOLOGICAL REVIEWS, MUNKSGAARD, XX, vol. 169, 1999, pages 31-44, XP008033666 ISSN: 0105-2896
- SZATMARI ISTVAN ET AL: "Activation of PPARgamma specifies a dendritic cell subtype capable of enhanced induction of iNKT cell expansion" IMMUNITY, vol. 21, no. 1, July 2004 (2004-07), pages 95-106, XP002307076 ISSN: 1074-7613
- AUGSTEIN P ET AL: "Surface and intracellular Fas expression associated with cytokine-induced apoptosis in rodent islet and insulinoma cells.", JOURNAL OF MOLECULAR ENDOCRINOLOGY APR 2003 LNKD- PUBMED:12683940, vol. 30, no. 2, April 2003 (2003-04), pages 163-171, ISSN: 0952-5041

## Description

The invention pertains to the field of immunology and immune therapy. More closely, the invention relates to manipulated professional antigen presenting cells having increased or decreased expression of a CD1 type I and II molecules, uses of PPARg (PPARgamma) modulators in the preparation of a pharmaceutical composition or kit for the treatment of a disease treatable by activation of CD1d restricted T-cells, e. g. in autoimmune diseases, allergies, post-transplant conditions or infectious diseases, or in the treatment of a neoplastic disease, e. g. skin cancer, hematological tumors, colorectal carcinoma, and therapeutic compositions and kits therefore. Furthermore, the invention relates to methods of manipulating professional antigen presenting cells and kits therefor.

BACKGROUND ART

The nuclear peroxisome proliferator-activated receptor (PPAR) is a nuclear hormone receptor family comprising three orphan receptors, PPARg (PPARgamma), PPARd (PPARdelta) and PPARa (PPARalpha) that are encoded by three different genes (Motojima, 1993) and are involved most probably in lipid uptake of the cells.

PPARg is a transcription factor that is activated by lipids, e. g. poly-unsaturated fatty acids and their metabolites, is known to be essential for fat cell formation, and is involved in regulation of genes of lipid uptake, accumulation and storage (Willson et al., 2001). It was cloned by different groups as a result of an effort to find transcription factors that regulate adipocyte-specific genes (Spiegelman, 1998, Willson et al., 2000 and Briggs, WO 99/05161), and was shown to be involved in the regulation of glucose homeostasis and to be linked to systemic insulin action [Spiegelman, Willson et al., see above].

PPARg is also part of a network of transcriptional regulators coordinately regulating lipid uptake and cholesterol efflux in macrophages by transcriptionally regulating CD36 and the oxysterol receptor LXRa (Liver X receptor-alpha). LXR in turn regulates the expression of multiple proteins involved in cholesterol efflux such as ABCA1 (Chawla et al., 2001b, Repa et al., 2000).

PPARg ligands have also been shown to mediate anti-inflammatory activities ill vitro and in vivo (Delerive et al., 2001; Desreumaux et al., 2001). Very recently it has been reported that treatment of NOD mice with PPARg ligands substantially reduced the development of type I diabetes (Augstein et al., 2003).

Various PPARg agonists have been suggested for use in the treatment of a number of disease conditions, e. g. in neoplastic diseases (Spiegelman et al., W09825598 ; Pershadsingh et al, WO 02/076177; Smith, US 6 294 559,2001 ; Evans et al, WO 98/29113) cutaneous disorders (Maignan et al., US 2003/0013734A1 ; Bemardon et al., US 2003/0134885A1), hyperglycemia and diseases associated with type II diabetes (Acton, WO 04/019869) and autoimmune disorders or other inflammatory conditions (Pershadshingh et al., US 6 028 088, 2000; Pershadshingh et al., WO 02/076177 ; Winiski, A, GB 2373725).

In 1998 it was demonstrated that ligands for PPARg were effective in reducing levels of inflammation (Ricote et al., 1998, Jiang et al., 1998). Moreover it was demonstrated by (Tontonoz et al., 1998, Nagy et al., 1998) that PPARg is involved in the conversion of monocytes to foam cells. These results suggested that endogenous PPARg ligands might be important regulators of gene expression during atherogenesis. However, it was assumed that these functions require costimulation of RXR or even other receptors (Spiegelman, 1998).

Later studies did not confirm former indications that PPARg might represent a target for anti-inflammatory therapy. Quite to the contrary, it was shown that PPARg expression is not essential for PPARg ligands to exert anti-inflammatory activity in macrophages and thus the receptor may be an inappropriate target for the development of anti-inflammatory drugs (Chawla et al., 2001).

Recently, Rosiglitazone (Avandia) and pioglitazone (Actos) have been approved for treatment of human type 2 diabetics. Recent PPARg agonist are under investigation. GI262570, a glitazone with potent glucose-lowering activity, which also reduces triglycerides and raises HDL cholesterol is currently in phase III clinical trial. A review on PPARg and its role in metabolic diseases is provided by Willson et al.[Annu. Rev. Biochem. 70, 341 (2001)].

Despite intensive research proposals for possible uses of PPAR ligands are partly controversial and the mechanism through which these ligands exert their effect has remained poorly understood. It was shown that PPARg is expressed in both macrophages (Tontonoz et al., 1998) and dendritic cells (DCs) (Faveeuw et al., 2000; Gosset et al., 2001; Nencioni et al., 2002) and that a key surface receptor associated with the uptake of apoptotic cells, the scavenger receptor CD36 (Albert et al., 1998) is known to be regulated by PPARg (Tontonoz et al., 1998), moreover the uptake of apoptotic cells by CD36 was shown to mediate a tolerogenic signal for DC (Urban et al., 2001).

To the best of present inventors knowledge, a possible role of PPAR modulators in manipulating professional APCs, e. g. DC or macrophages and therapeutic and research application coming therefrom, has not been suggested in the prior art. Thus, inventors were led to look at first into the potential role of PPARs in monocyte-derived DC differentiation.

Dendritic cells (DCs) are professional APCs, possessing a unique capacity to prime naive T cells. DCs are also key mediators of connecting innate and acquired immunity (Banchereau and Steinman, 1998). Importantly DCs are also believed to maintain sustained immunological tolerance to self macromolecules (Moser, 2003). Remarkably little is known of the transcriptional events controlling the differentiation and lineage commitment of DCs and their responses to external stimuli and/or internal signals. Myeloid DCs exist in two functionally distinct states, immature and mature. The capacity to internalize antigen is a property of immature DC (IDC). IDCs sample external antigens by macropinocytosis, phagocytosis, clathrin-mediated endocytosis and target them to MHC class II positive lysosomes (Sallusto et al., 1995). After detecting microbial products or pro-inflammatory cytokines, IDCs transform into mature DCs, with an exceptional capacity for T cell activation. This takes place in vivo in lymphoid tissues such as the lymph nodes and tonsils. Indeed, multiple subsets of DCs have been identified in these tissues (Summers et al., 2001). A major step of T cell activation is the presentation of processed peptides by MHC class II and/or class I membrane proteins expressed by professional APC, primarily DCs. DCs also have the capacity to present lipid antigens to specialized T cells by CD1 proteins. CD1 surface molecules represent a family of transmembrane glycoproteins expressed in association with beta-2-microglobulin on the APC membrane together with bound lipid or glycolipid ligands (Porcelli, 1995; Vincent et al., 2003). CD1 proteins are separated into two groups, group I (CD1a,-b, and-c) and group II (CD1d) molecules (Calabi et al., 1989). Group I CD1 proteins mediate specific T cell recognition of mycobacterial lipid and glycolipid antigens (Moody et al., 1999). CD1d can activate self-reactive CD1d-restricted T cells with features of activated/memory cells. A key CD1d-restricted T cell subtype is the autoreactive natural killer T (NKT) cell population, which is characterized by the invariant Valpha24 to Jalpha15 rearrangement preferentially in pair with Vbeta11 (iNKT cell) (Brossay et al., 1998). iNKT cells can be activated by alpha-galactosyl-ceramid (alpba-GalCer) in the context of CD1d (Kawano et al., 1997). The unique feature of iNKT cells is the rapid production of large quantities of IL-4 or IFNg, which are able to regulate both inflammatory and anti-inflammatory processes (Chen and Paul, 1997). It has also been suggested that tissue DC may be the target of self reactive CD1-restricted T cells, which are able to instruct DC maturation (Vincent et al., 2002). Interestingly, the lack of iNKT cell activation has been implicated in the development of autoimmune conditions (Hammond and Kronenberg, 2003) and iNKT cells were shown to be defective in Non-Obese Diabetic (NOD) mice, a model of autoimmune (type 1) diabetes (Carnaud et al., 2001; Kukreja et al., 2002). This suggests that NKT cells are intimately linked to sustaining immunological tolerance.

Monocytes can be differentiated ex vivo to DCs culturing them with GM-CSF and IL-4 (Sallusto and Lanzavecchia, 1994). The major source of IL-4 in peripheral tissues was attributed to iNKT cells, which are detected primarily in the liver, thymus, spleen and bone marrow (Kronenberg and Gapin, 2002). Monocytes are capable to differentiate into DCs in vivo after transendothelial transport (Randolph et al., 1998). Monocyte-to-DCs transition is greatly enhanced by a phagocytic stimulus (Randolph et al., 1999) suggesting that this process has an antigen-dependent component, whilst differentiation of DCs from other cell pools are believed to be steady state and cytokine driven (Liu, 2001). Nencioni and co-workers (Nencioni et al., 2002) disclose dendritic cells treated with peroxisome proliferator-activated receptor gamma (PPARg) agonists and expressing a decreased level of CD1a. However, there are clear indications in said paper that the PPARg activation of the dendritic cells of Nencioni severely blunted dendritic cell capacity to activate T lymphocyte proliferation and the secretion of the majority of cytokines, among other IL-10, IL-12 and IL-6 was blocked. Thus, dendritic cells of Nencioni can be considered a kind of immunologically impaired cells. Nencioni et al. is the closest prior art in respect of manipulated professional APCs. In WO98/29113 (Nagy et al., 1998) use of a number of PPARg agonists for the treatment of neoplastic diseases are taught. Said agonists are not used, however, to modulate professional antigen presenting cells, e.g. dendritic cells.

Having examined the role of PPARg in DC differentiation and function using human monocyte-derived DC, the present inventors surprisingly found that PPARg is immediately upregulated after the induction of the differentiation of DCs by IL-4 and GM-CSF. Activation of the PPARg receptor (and other PPAR receptors) altered the phenotype of DCs by changing the expression pattern of cell surface receptors involved in various functions and enhanced the internalizing activity of immature DCs. An increased expression of CD1d in DC by ligand activation of PPARg was unexpectedly detected, which led to the selective induction of CD1d restricted T cell, preferably iNKT cell proliferation, whereas CD1 type 1 molecules were down-regulated. PPAR antagonists elicited a nearly opposite effect. In addition PPARg was found to be inducible in blood myeloid DCs and present in interdigitating DCs in normal human lymphoid tissue in vivo. Thus, the lipid activated transcription factor, PPARg is involved in orchestrating a transcriptional response in differentiating monocyte-derived DCs leading to lineage specification and to the development of a DC subtype with increased internalizing capacity, efficient lipid presentation and the ability to induce iNKT cell proliferation. To sum up, it was found that upon PPAR modulator treatment in monocyte derived APC cells the expression pattern of certain cell surface molecules, in particular of CD1 gene family, as well as the cytokine secretion pattern altered, allowing a range of therapeutic applications to be developed.

BRIEF DESCRIPTION OF THE INVENTION

In a first aspect the invention relates to manipulated professional antigen presenting cell (APC) having increased expression of a CD1 type II molecule, preferably at least a CD1d molecule, relative to a control non manipulated cell, said APC being a dendritic cell (DC).

In the manipulated DCs of the invention preferably

the expression or activity of an endogeneous peroxisome proliferator activated receptor gamma (PPARg) is increased, i. e. the PPARg is upregulated, and/or

the expression of at least one of the following CD1 type I molecules is decreased, i. e. said molecules are downregulated: CD1a, CD1b and CD1c ; preferably at least the expression of CD1a is decreased, i. e. CD1a is down regulated.

The manipulated DC of the invention is preferably isolated.

The manipulated DCs of the invention are preferably obtainable by modulation of an endogeneous peroxisome proliferator activated receptor gamma (PPARg).

In a preferred embodiment the modulation is ligand-induced activation or increasing the expression. The modulation can be partial activation, increased expression or over expression, or a posttranslational modification increasing activity etc., too.

In a preferred embodiment, the expression of PPARg is increased relative to a control DC by gene transfection or either viral expression systems, e. g. retro-or lentiviral expression systems or by treatment with pharmacological agents.

The manipulated DC used in the invention may be a B-cell or a monocyte-derived cell, e. g. a monocyte, DC or macrophage.

The manipulated DC of the invention is, preferably a DC of myeloid origin, a monocyte derived DC or an interdigitating or plasmocytoid DC of lymphoid tissue, e.g. of tonsils.

Furthermore, the DCs according to the invention can be, as classified in humans: Langerhans cells (LC) of myeloid origin, which reside in the epidermis and in mucosal surfaces, myeloid interstitial and monocyte-derived DC also called as DC1 precursors, and/or plasmocytoid DC found in the blood and tonsils are considered as precursors of DC2 (Olweus et al, 1997)

Any of the above DCs or their precursors is applicable in the invention provided that, at least upon induction, they can express PPARg.

Dendritic cell precursors are e. g. monocytes, CD34+ progenitor cells, myeloid cells, in a preferred embodiment monocytes.

In a preferred embodiment the manipulated DC of the invention is a mature or an immature DC (MDC or IDC).

Preferably, the manipulated immature dendritic cells (IDCs) of the invention shows one or more of the following features relative to a non-manipulated control IDC:

an increased expression of CD86 expression,

an increased expression of HLA-DR expression,

a decreased expression of CD80,

a decreased expression of IL-12,

an increased expression of CD36,

no CD14 expression

an essentially unaltered DC-SIGN (CD209) expression,

an essentially unaltered CD206 (mannose receptor) expression.

Preferably, the manipulated DC of the invention has an increased endocytotic activity. Increased endocytosis may include an increased receptor mediated endocytosis, an increased phagocytosis or both, or any further type of endocytosis.

In a preferred embodiment endocytosis is phagocytosis, which may be detected by engulfment of latex beads.

Preferably, the manipulated mature dendritic cells (MDCs) of the invention shows an essentially unaltered T-lymphocyte stimulatory capacity relative to a non-manipulated control MDC. In an embodiment, in MDCs of the invention the CD80 marker is slightly down-regulated.

PPARg agonists are e. g. the following: modified fatty acids e. g. linoleic acid 9-hydroxy-octadecadienoic acid or 13-hydroxy-octadecadienoic acid or their ox-derivatives (9-HODE, 13-HODE, 9-oxoODE or 13-oxoODE) or e. g. 15-hydroxy-eicosatetraenoic acid (15-HETE) (components of occlude, natural ligands), or synthetic thiazolodinediones (TZD), e. g. troglitazone or rosiglitazone0 (RSG) or 15-deoxyA12, 14-PGJ2 (15dPGJ2) (synthetic ligands).

Various PPARg agonists have been disclosed in e. g. international publications W098/25598; W002/076177 ; W098/29113 ; W004/019869 ; W002/076177 ; US patents US 6 294 559; US 6 191 154 ; US 6 028 088 ; and published US patent applications US 2003/0013734A1 ; US 2003/0134885 and patents and publications referred therein.

In a further aspect the invention relates to any of the manipulated immature or mature DCs as defined above for use in the treatment of a subject in need of activated CD1d restricted T-cells, preferably CD1d restricted iNKT cells.

In an embodiment, the manipulated immature or mature DC is useful in autologous cell therapy.

In a further embodiment, the manipulated immature or mature DC of the invention is useful in the treatment of a tissue specific or systemic autoimmune diseases to induce tolerance e. g. in allergies, hypersensitivity reactions or post-transplant conditions, thereby helping graft acceptance, e.g. of corneal, cardiac, bone marrow, kidney, liver etc. allografts or xenografts.

Furthermore, the manipulated immature or mature DC of the invention is useful in the treatment of autoimmune diseases, e. g. type 1 diabetes, multiple sclerosis, autoimmune encephalomielitis, anterior chamber-associated immune deviation (ACID), lupus erithematosus, autoimmune hepatitis, inflammatory conditions etc.

In a further embodiment the manipulated immature or mature DC of the invention is useful for the treatment of neoplastic diseases, e. g. skin cancer, hematological tumors, colorectal carcinoma etc.

Preferably, the manipulated immature or mature DC of the invention is useful

-in cancer vaccination,

-to enhance the anti-tumor and anti-metastatic activity of IL-12, therefore the manipulated DC of the invention can be used in the treatment of a tumorous condition treatable by IFN-g and/or IL-12,

-to increase or facilitate natural tumor immunosurveillance, etc.

In a further embodiment the manipulated immature or mature DC of the invention is useful in the treatment of microbial infections, e. g. bacterial, parasitic, viral or fungal infections.

The manipulated mature DC of the invention is preferably capable of activating CD1d restricted T cell, preferably iNKT cells, if CD Id molecules are upregulated is said DCs.

The immature or mature DC is preferably a DC of any type as defined above.

In a further aspect the invention relates to use of PPARg agonists in the preparation of a pharmaceutical composition or kit for the treatment of a disease as defined herein.

In a preferred embodiment the disease is treatable by activation of CD1d restricted T-cells, preferably iNKT cells, e. g. autoimmune diseases (tissue specific and/or systemic), diseases requiring induction of tolerance, allergies, post-transplant conditions, infectious diseases.

In a preferred embodiment the disease is a neoplastic disease treatable by the activation of CD1d restricted T-cells, e. g. skin cancer, hematological tumors, colorectal carcinoma, melanoma, lymphoma, sarcoma, colon and breast cancer.

The disease treatable by the composition or kit is any of those specified above.

In a still further aspect the invention relates to a pharmaceutical composition or a pharmaceutical kit comprising a PPAR modulator and a CD1d ligand for use in therapy. The composition can be a composite type. The kit may comprise e.g. two compositions in a single unit package. In the pharmaceutical composition or in the pharmaceutical kit the PPARg modulator is a PPARg agonist for use in the treatment of a patient in need of activated CD1d restricted T-cells, preferably activated CD1d restricted iNKT cells.

According to a further preferred embodiment in the pharmaceutical composition or in the pharmaceutical kit the CD1d ligand is a self lipid antigen or an antigen having the same effect in the patient, wherein said patient is suffering from a condition which can be ameliorated by a tolerogenic or Th2 type immune response, e. g. an autoimmune disease.

According to a further preferred embodiment in the pharmaceutical composition or a pharmaceutical kit of claim the CD1d ligand is a foreign lipid antigen or an antigen having the same effect in the patient and wherein said patient is suffering from a condition ameliorated by an inflammatory or Th1 type immune response, e. g. a neoplastic disease.

Examples for foreign or self lipid antigens are described herein as well as in the art.

In a further aspect the invention relates to an immunological blood derived cell preparation comprising any of the manipulated DCs as defined above in a functionally active form. Preferably, in the immunological blood derived cell preparation the production of inflammatory cytokines, e. g. IL-12 and TNF is inhibited and the production of IL-18 is increased in the DC of the invention.

In a further aspect the invention relates to a kit for manipulating a DC

An embodiment is a kit for manipulating a in vitro, comprising at least the following

-a PPARg receptor agonist compound,

-means for isolating DC precursor cells,

-one or more reagent for detecting altered expression of CD1 type II molecules.

Preferably said kit is for inducing an increased or a decreased potential to CD1d or CD1a restricted T cells, preferably CD1 restricted iNKT cells

Furthermore a pharmaceutical kit is disclosed for autologuos cell therapy of a patient in need of manipulated professional APCs, comprising at least the following

-a PPARg receptor modulator compound,

-means for isolating APC precursor cells, e. g. blood monocyte cells or monocyte derived cells or a precursor thereof from a patient,

-one or more reagents for detecting altered expression of CD1 molecules

and

-means for administering the manipulated APC-s to the patient.

According to an embodiment the modulator is a PPARg agonist or activator, preferably a PPARg agonist. PPARg receptor agonists are taught above. Preferred agonists are synthetic thiazolodinediones (TZD), e. g. troglitazone or rosiglitazone (RSG) or 15-deoxyA12, 14-PGJ2.

Preferably, the means for detecting altered expression of CD1 molecules are specific labeled, preferably fluorescently labeled antigens.

In an embodiment, the CD1 molecules the expression of which is altered are CD1 type II molecules, preferably CD1d molecules. In an other embodiment the CD1 molecules the expression of which is altered are CD1 type I molecules, preferably CD1a molecules. Preferably, both type I and II CD1 molecules are modulated.

The expression of PPARg receptor in the cells may be altered by gene transfection or e. g. viral expression systems, preferably retro-or lentiviral expression systems or by treatment with pharmacological agents.

Examples for precursor cell useful in the kits of the invention are given above.

There is also disclosed an assay method for testing PPARg agonists for their effect on DCs to activate NKT cell *characterized by*

-isolation of DCs or their precursors

-ligand-induced activation of endogeneous peroxisome proliferator activated receptor gamma (PPARg) in DC or in their precursors

-differentiation of treated DC or of their treated precursors to mature DC

-assessing the altered surface marker pattern of the DC and optionally,

-assessing whether the manipulated DC has an increased potential to activate NKT cells. Preferably, the altered surface marker pattern includes an increase in CD1d expression.

In a further aspect the invention relates to a method for manipulating DCs or their precursors to induce increased expression of CD1 type II molecules to influence CD1 type II restricted T cell, preferably CD1d restricted iNKT cell activities, said method comprising

-ligand-induced activation or increasing expression of PPARg, in the precursor,

-differentiation of treated DC or of their treated precursors to immature or mature DC,

wherein the manipulated DC has an increased potential to activate CD1 type II restricted T cells.

In a preferred embodiment the increased expression is achieved by enforced expression of PPARg in DC by gene transfection, viral expression systems (retro-or lentiviral) or by treatment with pharmacological agents.

Preferably the ligand-induced activation of PPARg is induced in blood monocytes.

In a preferred embodiment, the ligand is a PPARg agonist.

In a preferred method the DC precursor cell is isolated and ligand-induced activation of PPARg is induced by ex vivo treatment, wherein the precursor cell is selected from monocytes, CD34+ stem cells or blood-derived DC precursors.

In an alternative method, the ligand-induced activation of PPARg is induced by in vivo targeting of the DC precursors.

In the above methods the ligand-induced activation of PPARg can be effected preferably by a synthetic thiazolodinedione (TZD), e. g. rosiglitazone, or by modified fatty acids, e. g. by linoleic acid 9-HODE and 13-HODE, hydroxy-octadecadienoic acid.

The DC precursors are preferably any of the DC precursors taught herein, e. g. monocytes, CD34+ progenitor cells, etc.

In a further aspect the invention discloses a method of autologous cell therapy in a patient, which comprises

-isolation of an DC precursor

-ligand-induced modulation of PPARg in the DC precursors,

-differentiation of treated DC or DC precursor to immature or mature cells.

An embodiment of the method for autologous cell therapy comprises

-isolation of an DC precursor

-ligand-induced activation of PPARg or enhancing PPARg expression by gene transfer with PPARg expression vectors in DC precursors

-differentiation of treated DC or DC precursor to immature or mature cells,

-reintroducing the differentiated cells into the patient,

wherein the treated DCs are capable of NKT cell activation.

DEFINITIONS AND ABBREVIATIONS

'CD1d-restricted T cells'are self-reactive T cells which can be activated by CD1d, said cells having features of activated/memory cells. A key CD1d-restricted T cell subtype is the autoreactive natural killer T (NKT) cell population, which is characterized by the invariant Valpha24 to Jalpha15 rearrangement (iNKT cell) preferably in pair with Vbeta11 (Brossay et al., 1998). iNKT cells preferably can be activated by alpha-galactosyl-ceramid (alpha-GalCer) in the context of CD1d (Kawano et al., 1997).

The term '*agonist*'*,* as used herein, refers to a molecule which, when interacting with an biologically active molecule, causes a change (e.g., enhancement) in the biologically active molecule, which modulates the activity of the biologically active molecule. For example, agonists can alter the activity of gene transcription by interacting with RNA polymerase directly or through a transcription factor or signal transduction pathway.

As used herein, the term'*PPARg agonist'* refers to a compound or composition, which, when combined with PPARg, directly or indirectly stimulates or increases an *in vivo* or *in vitro* reaction typical for the receptor (e.g., transcriptional regulation activity). The present invention contemplates that any known or future identified PPARg agonist will find use with the present invention.

The terms *'antagonist'* or *'inhibitor'*, as used herein, relate to a molecule which, when interacting with a biologically active molecule, blocks or modulates the biological activity of the biologically active molecule. Inhibitors and antagonists can effect the biology of entire cells, organs, or organisms (e.g. an inhibitor that slows or prevents neuronal degeneration and death).

The term '*modulate*', as used herein, refers to a change in the biological activity of a biologically active molecule. Modulation can be an increase or a decrease in activity, a change in binding characteristics, or any other change in the biological, functional, or immunological properties of biologically active molecules.

An '*increase or a decrease'* in activity, expression, concentration etc. refers to a change which is detectable under the given conditions, considering the statistical error or the signal/noise ratio of the given measurement.

Reference herein to '*a level and lorfunctional activity*' in the context of a protein produced by a specified cell is to be taken in its broadest sense and includes a level and/or functional activity of the protein that is produced in a single cell or in a plurality or population of cells. In the latter case, therefore, it will be understood that the phrase will encompass a mean level and/or functional activity of the protein produced by a plurality or population of cells.

By '*autologous' is* meant something (e. g. cells, tissues etc.) derived from the same organism.

By '*isolated*' is meant material that is substantially or essentially free from components that normally accompany it in its native state.

The term '*patient*' refers to patients of mammalian, especially human, or other animal origin and includes any individual it is desired to examine or treat using the methods of the invention. However, it will be understood that *'patient'* does not imply that symptoms are present. Suitable animals that fall within the scope of the invention include, but are not restricted to, primates, livestock animals (e. g. sheep, cows, horses, donkeys, pigs), laboratory test animals (e. g. rabbits, mice, rats, guinea pigs, hamsters), companion animals (e. g. cats, dogs) and captive wild animals (e. g. foxes, deer, dingoes, reptiles, avians, fish).
The following abbreviations are used herein:

alphaGalCer: alpha-galactosyl-ceramide

APC: antigen presenting cell

CD: cluster of differentiation

DC:dendritic cell

ELISA: enzyme-linked immunoassay

ELISPOT: enzyme-linked enzyme spot assay

FABP4: fatty acid binding protein

G-CSF: macrophage colony stimulatory factor

GM-CSF: granulocyte-macrophage colony stimulatory factor

IL: interleukin

IDC: immature dendritic cell

LC: Langerhans cell

MCM: macrophage conditioned medium

MDC: mature dendritic cell

MHC: major histocompatibility complex

NK: natural killer

Q-RT-PCR: quantitative reverse transcription polymerase chain reaction

PPAR: peroxisome proliferator-activated receptor

RSG: Rosiglitazone

SCF: stem cell factor

TA: tumor antigen

TAA: tumor-associated antigen

TCR: T cell receptor

Th: T helper

TNF: tumor necrosis factor

BRIEF DESCRIPTION OF THE FIGURES

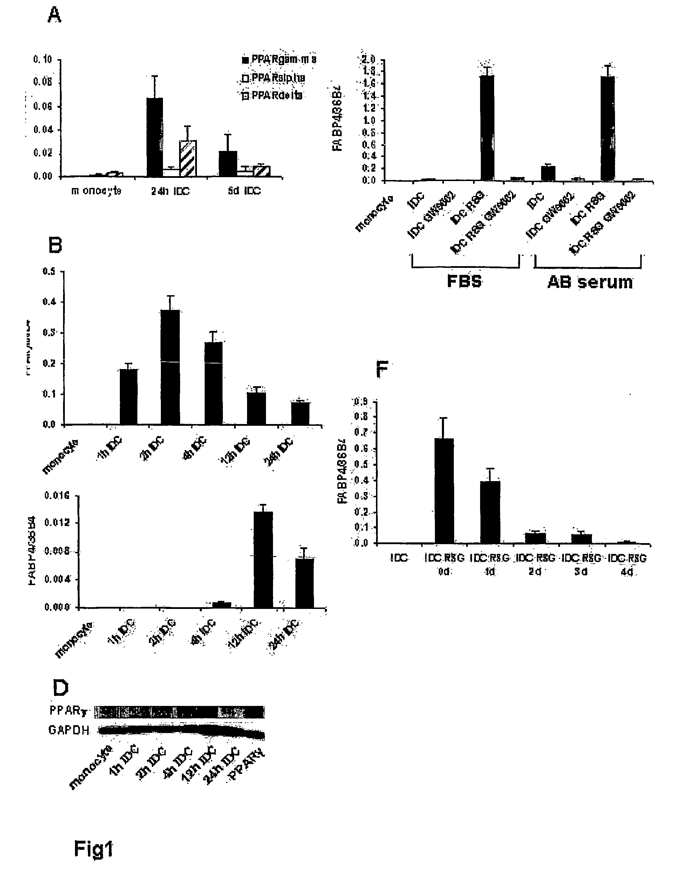

Figure 1. The expression of PPARg very rapidly reaches high levels during DC differentiation and can be activated by PPARg specific agonist.

(A) Monocytes were cultured for 24 hours or 5 days in the presence of 500 U/ml IL-4 and 800 U/ml GM-CSF for the generation ofIDCs. The mRNA levels for PPARs were determined by real-time quantitative RT-PCR as described in experimental procedures. Data are expressed as a ratio of the PPAR transcripts relative to 36B4 expression. Data show the average expression and standard deviation of 5 independent experiments.

(B, C) Kinetics of PPARg and FABP4 expression. Cells were harvested at the indicated points of time and the mRNA expression was determined by RT-PCR. Data are expressed as a ratio of the PPAR or FABP4 transcripts relative to 36B4 expression. Error bars indicate the standard deviation of the relative expression.

(D) Western blot analysis of PPARg protein expression in differentiating DC. 20 microg of extract from the indicated cell types were separated by SDS-PAGE, transferred to PDVF membrane, and subjected to Western blot analysis using rabbit anti-PPARg (H-100) and murine anti GAPDH antibodies. The identity of the 55 kDa band was confirmed by co-migration with a band seen in the cell extract of a PPARg transduced 293 cells.

(E) Determination of the transcript levels of FABP4 of vehicle or 2.5 microM Rosiglitazone (RSG) treated DC in the presence or absence of 5 microM GW9662. Cells were cultured using 10% FBS (fetal bovine serum) or FBS was replaced with human AB serum.

(F) Kinetics of FABP4 expression. IDCs were harvested and analyzed on day 5. The ligand treatment (2.5 microM RSG) was performed at the indicated points of time.

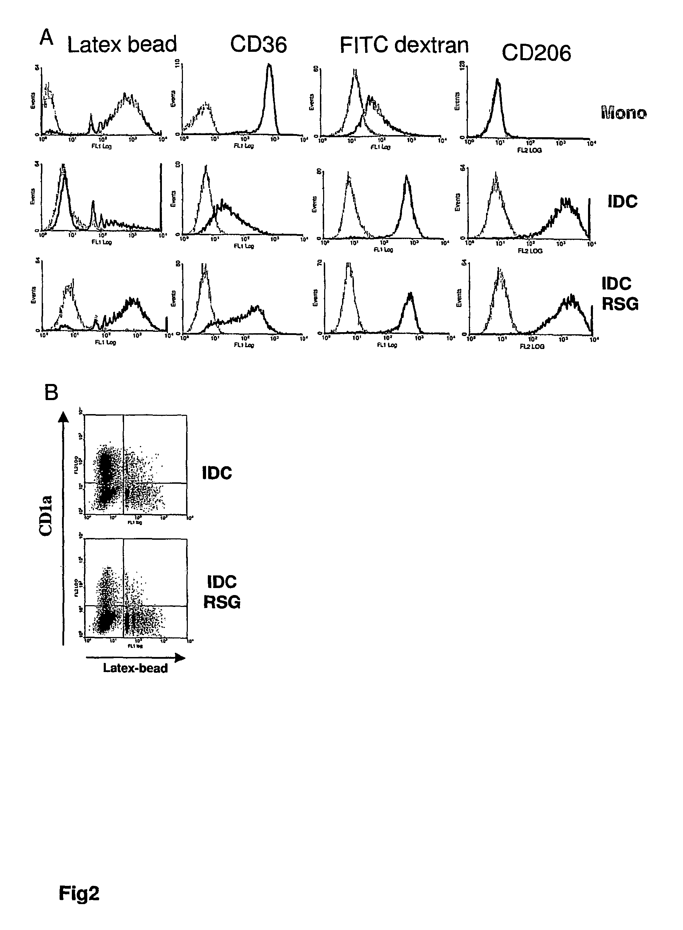

Figure 2. Activation of PPARg enhances the phagocytic activity of IDCs.

(A) Monocytes (Mono) were cultured for 5 days as described in experimental procedures in the presence (IDC RSG) or the absence (IDC) of Rosiglitazone (2.5 microM). The phagocytic capacity of DC was evaluated by measuring the uptake of FITC-dextran or latex-bead (24h incubation) as described experimental procedures.
Cell surface expression of CD36 and CD206 was determined by flow cytometry. Cell surface receptor specific mAb indicated (solid line) vs isotype matched control (dotted line).

(B) Latex bead uptake (4h incubation) of the CD1a+ and CD1a-DC was measured by flow cytometry.

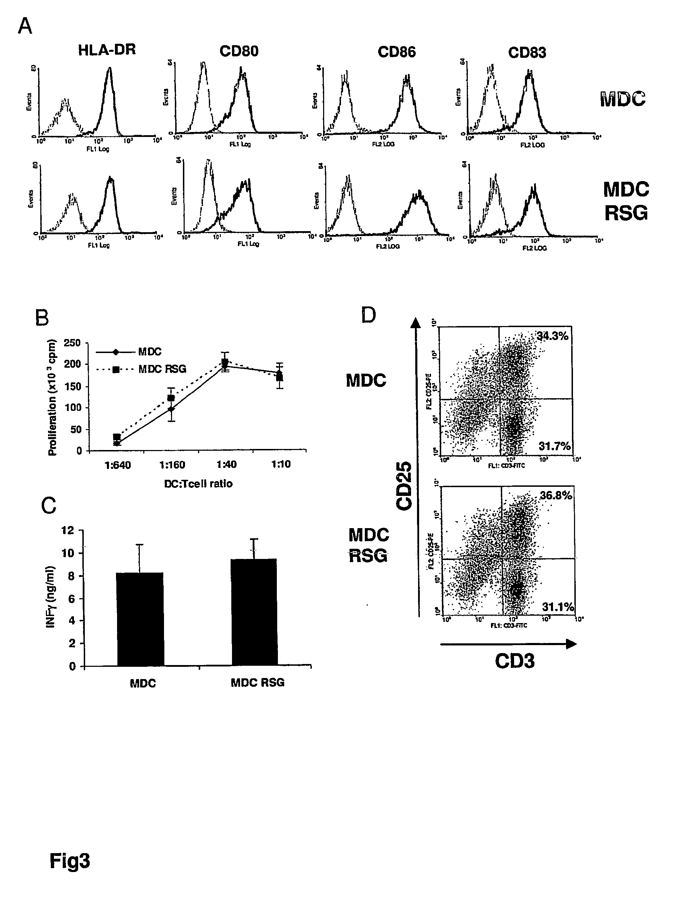

Figure 3. PPARg treatment does not interfere with DCs maturation.

Monocytes (Mono) were cultured for 6 days as described in experimental procedures for the generation of MDC.

(A). Phenotypic characterization of MDC. MDC were stained with mAbs indicated in the figure. Cell surface molecule specific mAb indicated (solid line) vs isotype matched control (dotted line).

(B) MDC or RSG treated MDC (2.5 microM) were cocultured with allogeneic PBMC (2×10⁵/well) at ratios ranging between 1: 10 to 1: 640 (DC: T cells) for 5 days. [H] -thymidine (1 microCi/well) was added for the last 16 h of the cultures, the cells were harvested, and proliferation was measured by detecting [H]-thymidine incorporation by a scintillation counter.

(C) MDC or RSG treated MDC (2.5 microM) were cocultured with allogeneic PBMC (2×105/well) at 1: 10 DC-T cell ratio. Supernatants were collected at day 5 and were assayed by INFg ELISA. Results are represented as mean and SD of 4 independent experiments.

(D) MDC or RSG treated MDC (2. 5 microM) were cocultured with allogeneic PBMC (2×1O5/well) at 1: 10 DC-T cell ratio. PBMCs were harvested at day 5 and analyzed by FACS. Cells were stained by two-color flow cytometry using the indicated T-cell specific Abs.

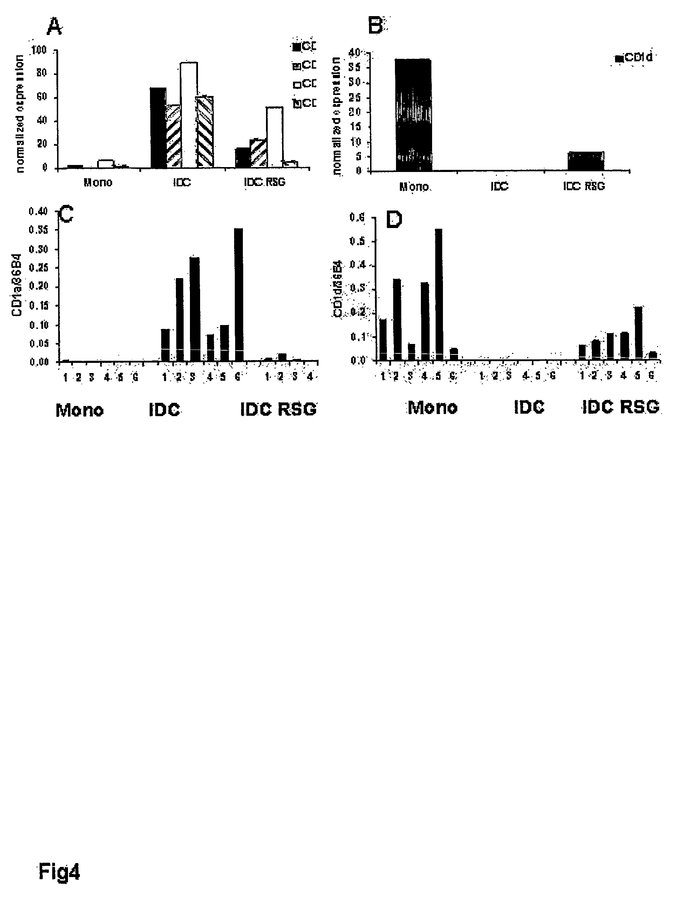

Figure 4. PPARg activated DCs express more CD1d and less CD1a than non-treated cells.

Monocytes (Mono) were cultured for 5 days as described in experimental procedures in the presence (IDC RSG) or the absence (IDC) of 2.5 microM Rosiglitazone.

(A, B) The relative expression of group I CD1 s and CD1d (fig A and B respectively) were assessed by using affymetrix microarray data as described in experimental procedures.

(C, D) The mRNA level of CD1a and CD1d were also determined by RT-PCR. RT-PCR analyses of 6 independent experiments are shown.

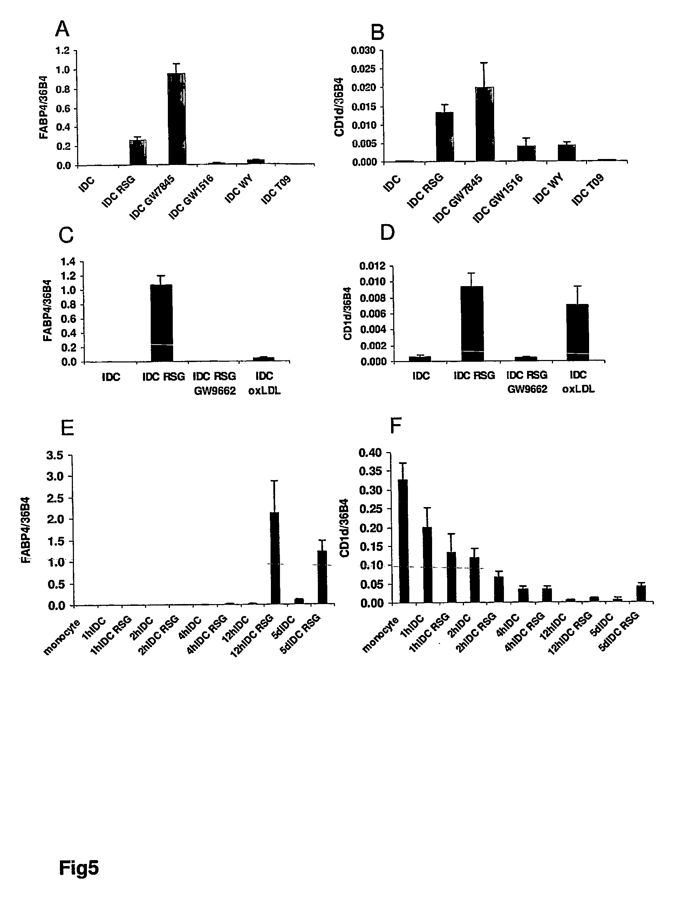

Figure 5. PPARg activated DCs express more CD1d and less CD1a than non-treated cells. Data are expressed as a ratio of the FABP4 or CD1d transcripts relative to 36B4 expression. Error bars indicate the standard deviation of the relative expression.

(A, B) Transcript levels of FABP4 and CD1d (A and B, respectively) were determined in IDC treated with various ligands: 2.5 microM Rosiglitazone (RSG), 200 nM GW7845, 1 microM GW501516,20 microM WY14643 and 1 microM T0901317.

(C, D) Determination of FABP4 and CD1d mRNA levels (C and D, respectively) in IDC treated with 2.5 microM RSG, in the presence or absence of 5 microM GW9662.
Cells were also treated with 20 microg/ml oxidized LDL.

(E, F) Kinetics of FABP4 and CD1d expression during IDC differentiation. Cells were harvested at the indicated points of time and the mRNA expression was determined by RT-PCR.

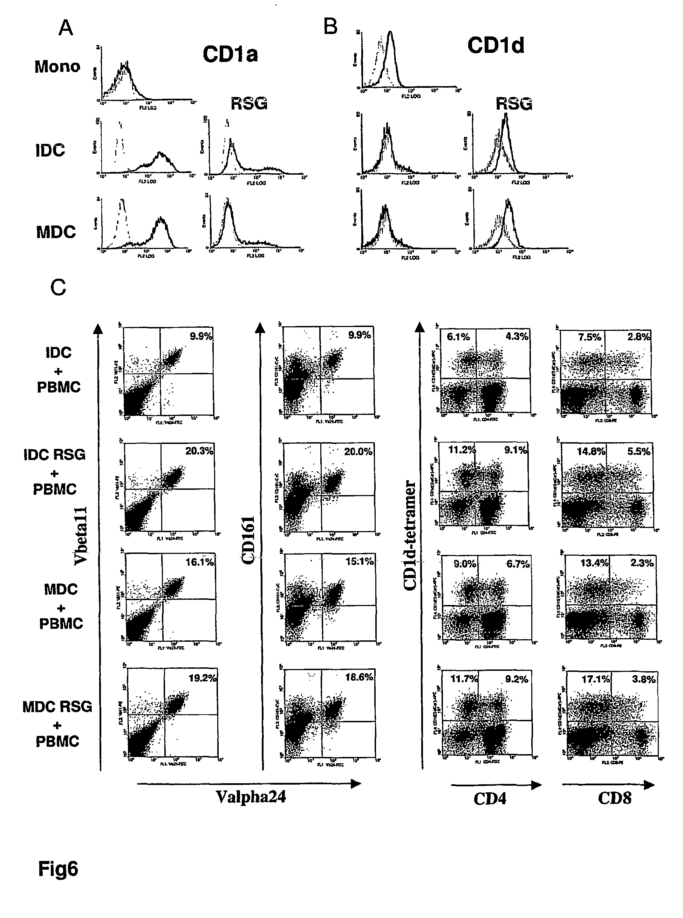

Figure 6. PPARg activated DCs express more DC1d and less CD1a than their untreated counterparts and promote the expansion of iNKT cells.

(A, B) Monocytes (Mono) were cultured for 5 days for obtaining IDC or for 6 days for MDC as described in experimental procedures. Cells were treated with 2.5 microM RSG. Monocytes and DCs were harvested and stained with CD1a or CD1d specific mAbs (A and B, respectively). Cell surface specific mAb indicated (solid line) vs. isotype control (dotted line).

(C) alpha-GalCer-pulsed and RSG treated (2.5 microM) or untreated IDC or MDC were cocultured with autologous PBMC for 5 days, harvested and the cells were stained by two-color flow cytometry using the indicated iNKT, T-cell specific Abs and CD1d-tetramer.

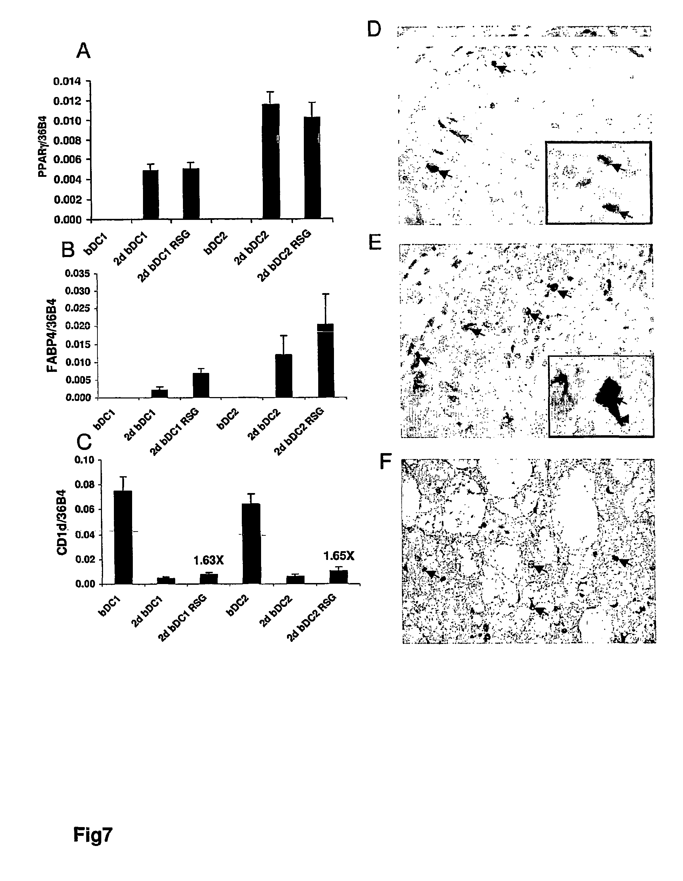

Figure 7. PPARg is expressed in blood DC and in interdigitating DCs of human tonsils

(A, B, C) Transcript levels of PPARg FABP4 and CD1d (A, B and C, respectively) were determined in freshly isolated myeloid blood DC (BDCA1) or BDCA1 cell cultured for 2 days in RPMI 1640 without any cytokine treatment. Data are expressed as a ratio of PPARg, FABP4 or CD1d transcripts relative to 36B4 expression. Error bars indicate the standard deviation of the relative expression.

(D, E, F) Expression of PPARg and S-100 in lymphoid tissue.

(D) Nuclear expression of PPARg protein in cells (arrow) located in the surface epithelium of normal human tonsil (40x). PPARg-positive cells were also found surrounded by lymphoid cells in the perifollicular zones of the tonsil (Inset, 100x).

(E), Similar field as shown on (D) exhibits numerous S-100 positive DCs in the surface epithelium of the tonsil (40X). Inset (100x) : double positive cells can be found in the lymphoid compartments which co-expressed the nuclear PPARg (arrow) and the cytoplasmic S-100 protein (arrowhead).

(F) Nuclear expression of PPARg in human brown fat cells (arrows) obtained from hybernoma.

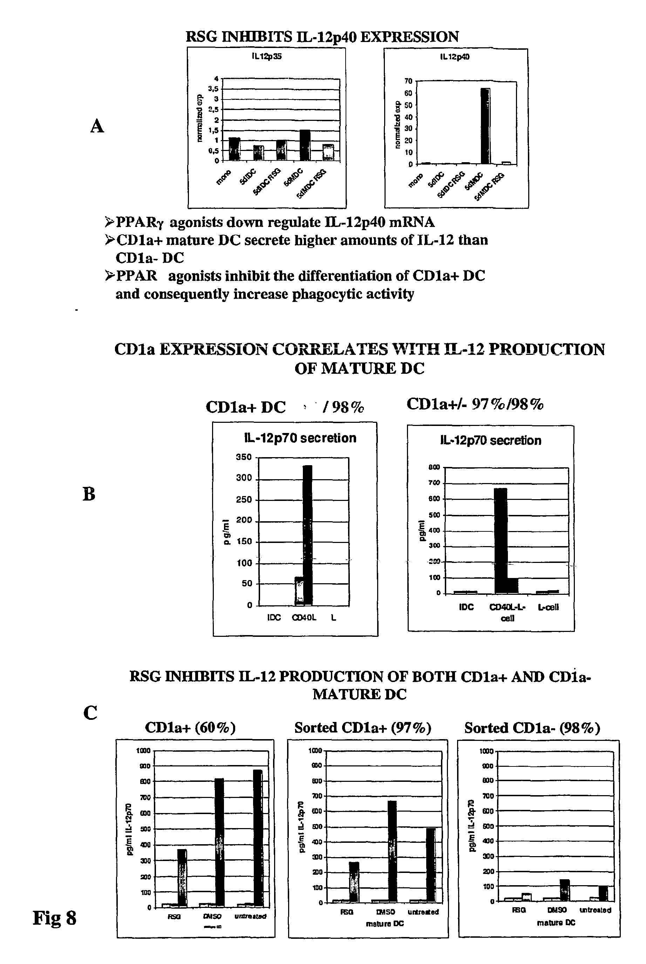

Figure 8. IL 12 expression in RSG treated and non-treated cells

(A) IL-12p35 and IL-12p40 expression by Monocites and in RSG treated and non-treated IDC and MDC after 5 days differentiation

(B) CD1a expression and IL-12 production in IDCs and MDCs of various phenotype.

(C) IL-12 production in RSG treated and non-treated mature DCs comprising mainly CD1a+ or CD1a-phenotype.

DETAILED DESCRIPTION OF THE INVENTION

Below the invention is disclosed in more detail with reference to certain examples.

Dendritic cells originate from various precursors and depending on their origin and tissue environment they give rise to various functionally distinct cell types (Liu, 2001).

In an embodiment of the invention, monocyte-derived DCs are used. Monocyte-derived DCs represent an in vivo relevant cell type generated from blood monocytes undergoing antigen-dependent differentiation to become fully functional DCs (Randolph et al., 1998).

According to further embodiments DCs of myeloid origin, e. g. Langerhans cells or interstitial cells, or plasmocytoid DCs are applicable, or, according to an other classification, blood DCs or DCs of lymphoid organs can be used since both are capable of expressing PPARg. This suggests that a wide range of DCs can be useful in the present invention.

DC activation is linked with marked functional changes and results in the loss of endocytic/phagocytic receptors, upregulation of adhesion, co-stimulatory molecules and chemokine receptors, a change in morphology, expression of chemokine receptors and mobility, reorganization of lysosomal and MHC class 11-rich intracellular compartments, and release of cytokines and chemokines.

At first the present inventors characterized the profile of PPARg mRNA induction it was found that it was already at high levels after one hour of DC differentiation. It peaks around two hours, but remains at relatively high levels throughout the course of differentiation. We also determined the PPARg protein and responsiveness of differentiating DC by measuring target gene expression and found that both parameters were matching that of the peak of receptor mRNA expression levels. These findings establish PPARg as an immediate early transcription factor in monocyte-derived DC differentiation that brings about increased PPARg responsiveness at a very early stage of DC differentiation.

By using receptor-specific antagonist we also provided evidence for a small, but detectable level of endogenous ligand activity stimulating this receptor. These data clearly show that DCs are primed for PPARg ligand activation in the first 24 hours of differentiation. Once the receptor got activated by endogenously produced or exogenously provided ligands it leads to altered gene expression. Though it is not well understood what the source of ligand could be, it is known that 15-lipoxygenase (15-LO) produces 15-HETE or 13-HODE, which are ligands or activators of PPARg (Huang et al., 1999). Interestingly 15-LO is also induced upon DC differentiation (Spanbroek et al., 2001 data not shown). Another (extracellular) source could be oxidized LDL containing 9-and 13-HODE (Nagy et al., 1998). Oxidized LDL indeed could modulate DC differentiation from monocytes (Perrin-Cocon et al., 2001) and our data indicated that oxLDL was able to induce the expression of a PPARg target gene in DCs. It is also possible that cell debris and/or apoptotic cells contain lipid activators of PPARg and therefore digestion of engulfed particles would lead to the release of ligands or activators.

It has been shown previously that differentiating DCs express the nuclear hormone receptor PPARg (Gosset et al., 2001; Nencioni et al., 2002). The results obtained were mostly inhibitory and suggested that activation of the receptor inhibited the immunogenicity and cytokine production of DC. The present inventors confirmed some of these earlier data on the induction of PPARg and the phenotype of DCs, but surprisingly obtained different results regarding the T cell activating potential of PPARg activated DCs. The reason for the differences may be the usage of different conditions and more importantly different doses of ligands. It has been shown that higher doses of PPARg activators may induce receptor independent inhibitory effects (Chawla et al., 2001a).

Using global gene expression profiling, we also found several positive responses which led us to the conclusion that PPARg is not simply an inhibitor of the antigen presenting functions of DCs but rather modulated their phenotypic features and functional activities leading to the development of a DC subset.

By systematically characterizing changes of the gene expression pattern induced by PPARg we were able to uncover more precisely the phenotype of DC induced by receptor activation. This appears to be a specification of a subset of DCs rather than inhibition of differentiation per se as suggested by others (Gosset et al., 2001 ; Nencioni et al., 2002). This can be attributed to the generation of fully functional and potent immature DC with increased internalizing capacity as well as a characteristic CD1 expression pattern. Most importantly we did not observe differences in the T cell stimulatory activity of MDC. This underscores the notion that the uptake and presentation of antigen may be altered, but the T-cell activating potential including the expression of co-stimulatory molecules does not change.

The immature DC phenotype induced by IL-4 and GM-CSF is CD14-CD209+, CD1a++, CDId-, CD86 +, CD80++, CD36+, this is converted by PPARg activation into a CD14-CD209+, CD1a-, CD1d+, CD86++, CD80+, CD36++ phenotype. A similar cell-surface pattern was reported previously (Nencioni et al., 2002). This phenotype, together with inhibited IL-12 expression (F aveeuw et al., 2000; Gosset et al., 2001) and increased CD86/CD80 ratio, resembles tolerogenic DC but with increased phagocytic activity.

These finding led the inventors to raise the question if PPARg-induced DCs are tolerogenic rather than inflammatory and are specialized for engulfing and presenting tissue-derived lipids for autoreactive NKT cells, which are known to have access to peripheral tissues and produce high amounts of anti-inflammatory IL-4 in the absence of microbial antigens (Vincent et al., 2002). These questions can be best answered in an in vivo setting.

The present inventors think that PPARg, being a lipid activated transcription factor (Willson et al., 2001), is ideally suited to coordinate lipid uptake and presentation. Its' regulation of CD36 is well established (Nagy et al., 1998), the pivotal role of CD36 in dendritic cell biology (Urban et al., 2001) and increasing CD1d mRNA and protein expression all suggest a coordinated set of events involving lipid uptake and presentation. According to our knowledge PPARg is found to be the first transcription factor implicated in antigen uptake and presentation. Thus, it was concluded that there might be a link between the lipid molecules activating PPARg and being presented by CD1d.

Very little is known of the transcriptional regulation of CD1 genes. Group I CD1 proteins are inducible on human monocytes by in vitro exposure to GM-CSF or GM-CSF and IL-4 (Kasinrerk et al., 1993; Porcelli et al., 1992) or in vivo after pathogen exposure (Sieling et al., 1999). Our microarray data indicated that group I CD1s are coordinately upregulated during monocyte-derived DC differentiation. It was described that RSG treated DC express less CD1a (Nencioni et al., 2002). We confirmed this finding and extended with results showing that the mRNA level of CD1a in ligand treated cells was also lower than in control cells. We also found that the activation of PPARg diminished the expression of the whole group I CD1s during DC differentiation. The mechanism of this coordinated decreased expression is unknown.

More closely, CD1a+ positive DC, e. g. mDC produce IL-12 upon CD40 ligation, whereas CD1a-immature DC are more active in phagocytosis but after maturation they do not produce IL-12. The ratio of CD1a+ and CD1a-DC can be manipulated by the ligand-induced activation of PPARg (Figures 9 and 10). We suggest that the expression of CD1a on mDC may influence the outcome of DC therapy upon regulation by exo/endogenous lipids present in the serum and/or induced by the activation of PPARy, known to be involved in the regulation of lipid metabolism.

Interestingly, CD1d is not up-regulated in GM-CSF and IL-4 treated human monocytes in vitro under the same conditions that up-regulates group I CD1s (Exley et al., 2000). Our data, however, shows that CD1d is down regulated during the course of DC differentiation from monocytes, but PPARg activated DC induced their CD1d expression after an initial decrease. The effect of PPARg on CD1d expression may be an indirect effect, since a longer exposure time (12h) was needed for the increased CD1d expression. Nonetheless we clearly showed that this is a receptor dependent process. Without being bound by theory, we believe that CD1d regulation in ligand treated IDCs is a biphasic process. There is an initial downregulation not influenced by PPARg activation followed by a PPARg-dependent upregulation.

CD1d expression-is indispensable for the generation and expansion of iNKT cells. CD1 d is shuttling from the cell surface to late endosomes and MHCII compartments, which facilitates loading of both endogenous and exogenous lipids and their presentation for CD1d restricted T cells (Moody and Porcelli, 2003). We detected an increased frequency of the rare iNKT cells if we co-cultured autologous PBMC with RSG treated and alpha-GalCer-loaded IDC. By characterizing the induced iNKT cell population we found that it includes both CD161 positive and negative cells and has a similar composition of DN, CD4+ and CD8+ cells as the control population. The specificity and CD1d restriction of these cells, proven by CD1d tetramer staining confirmed the increased lipid presenting capacity of RSG treated IDC.

Thus, the cytokine pattern produced by sorted NKT cells or autologous T-lymphocytes, activated in the presence of untreated or RSG-treated DC, suggests a shift to an anti-inflammatory response induced by PPARg activation in maturing DC, characterized by high IL-10 but low IL-12 production (fig 8). We suggest that the mutual interaction of RSG-treated DC and activated iNKT cells enables to regulate T-cell responses by modifying cytokine production and a subsequent T-cell polarization.

There are multiple implications of the pathways discovered. First, this provides a clear insight into the regulatory logic of dendritic cell lineage specification. It shows how extracellular signals can influence lineage specification and gene expression. Second, it also provides an entry point for intervention into this process in vivo by modulating CD1d expression and thereby iNKT cell activation. Third, there are clear biological consequences of increased CD1d expression and NKT cell activation.

There are in vivo examples of CD1d-NKT cell dependent processes such as the NOD mouse. In this model the expansion and differentiation of autoimmune effector T cells leads to beta-cell destruction and the development of type 1 diabetes. This process has been tied to both the CD1d locus and iNKT cells (Carnaud et al., 2001; Shi et al., 2001; Wang et al., 2001). The deletion of the CD1 locus contributes to disease progression and alphaGalCer treatment results in amelioration of the disease (Sharif et al., 2001).

Moreover, for certain autoimmune diseases such as type I diabetes and multiple sclerosis it is known that a normal function of otherwise less functional iNKT cells can be restored by alphaGalCer.

In other autoimmune conditions, e. g. systemic lupus erithematosus, systemic sclerosis, colitis, psoriasis, rheumatoid arthritis, Sjogren syndrome, polymyositis or autoimmune hepatitis, lower iNKT cell numbers have been reported (Wilson et al., 2003). The effect of alphaGalCer is questionable in some of these conditions since it may elicit an IFN-g driven response (Thl-like response) possibly leading to exacerbation of the disease. Analogues, at the same time, such as OCH may have diverse effect and may elicit a predominantly IL-4 response. Thus, careful selection of CD1d ligand and study of the induced response is necessary.

In any way, the manipulated DCs of the invention having the advantageous properties disclosed herein, may well be used in a condition characterized by reduced number or activity of iNKT cells (CD1d restricted T cells) to expand said cells. In the knowledge of the pertinent art and of the present disclosure the skilled person has to and will be able to decide on the question what kind of, if any, additional CD1d ligand has to be administered.

It is assumed that in general, wherein a foreign microbial lipid or a lipopeptide antigen is used as a CD1d ligand, the immune response mediated by the activated iNKT cells can be pro-inflammatory (aggressive) with a relatively high probability. Therefore if the manipulated APC or DC of the present invention is used together with such an antigen, an antitumor immune response may be elicited.

Examples of foreign lipid antigens include e. g. mycolyl lipids, e. g. mycolic acids or derivatives, e. g. esthers thereof, e. g. glucose monomycolate, acyl-glycerols, e. g. diacylglycerols, e. g. phosphatidylinositol or phosphatidyl-inositol dimannoside, poly-isoprenoid lipids, e. g. mannosyl-beta-1 phosphoisoprenoid, lipopeptides an example of which is didehydroxy mycobactin or, preferably, sphyngolipids, e. g. sulfatide or alpha-galactosyl-ceramide (alpbaGalCer). However, a part of them may not be a ligand for CD1d, therefore in a particular application the effect of the given antigen should be carefully checked.

It has been shown that CD1d-restricted T cells can be stimulated by exposure to CD1d expressing APCs in the absence of foreign antigens, as well (Exley et al, 1997). The present inventors found that immature or mature DCs in which expression of CD1d is upregulated is capable of activating CD1d restricted T cells. In this case the immune response elicited by these T cells, preferably SINKT cells is most probably rather tolerogenic than inflammatory.

Examples for self antigens include ubiquitous phospholipids, e. g. phosphatidylinositol, phosphatidylethanolamine or phosphatidylglycerol, self-sphingolipids, gangliosides e. g. GD3 or mammalian ceramides. It is proposed according to an embodiment of the invention to use these self antigens or analogues thereof together with the manipulated DCs (or APCs) or with a PPAR-agonist, if a tolerizing effect via the activated CD1d restricted T-cells is desired, e. g. during certain autoimmune or inflammatory conditions.

Certain foreign antigens in certain conditions or diseases may elicit or restore tolerance which is most probably a question of the structure of said antigen. Known examples are e. g. the treatment of type I diabetes or multiple sclerosis by alphaGalCer, as mentioned above.

Thus, the invention provides for a screening method for identifying a CD1d ligand capable of activating CD1d-restricted T cells eliciting a tolerogenic or suppressive immune response, wherein a manipulated APC of the invention, e. g. a DC in which CD1d expression is upregulated, is contacted with a candidate compound in an environment comprising CD1d-restricted T cells and assessing whether said T cells have been activated and/or a tolerogenic or suppressive immune response or cytokine response is evolved. The response can be observed either in vivo or in vitro if the sample comprises the appropriate cells and factors enabling the response. If such a response is observed, the candidate compound is considered as a CD1d ligand capable of activating CD1d-restricted T cells. Proposed candidate compounds are known self lipid or peptide-lipid or liposaccharide antigens.

As an example, ifIL-12 or IL-10 levels and, possibly in turn, Th1 cytokines, e. g. IFN-g level is elevated, the immune response will be probably aggressive (inflammatory), if the levels of these cytokines is decreased and/or the level of Th2 cytokines, e. g. IL-13, IL-10 or IL-4 is elevated the resulted immune response will be probably suppressive (tolerogenic).

According to an embodiment of the invention there is a possibility to upregulate CD1 type 1 molecules, e. g. CD1a by a PPARg antagonist or by a method having analogous effect. This elevates e. g. IL-12 levels which enables a pro-inflammatory immune response. This can be utilized e. g. in cancer therapy. In an advantageous embodiment a PPARg agonist and an antagonist effect can be used sequentially. In the phase when, due to the PPARg agonist effect, CD1d is upregulated, CD1d restricted T cells can be rapidly activated preferably by addition of a foreign lipid or analogous antigen, so as to elicit possibly a pro-inflammatory response. In the other phase, due to the PPARg antagonist effect, CD1 type 1, e. g. CD la molecules are upregulated and inflammatory cytokines are secreted by the DC to support and maintain Th1 differentiation. Thereby, if the two phases, i. e. the agonist and antagonist effects are suitably combined, an enhanced inflammatory effect may be achieved. Thereby a condition, e. g. a cancer can be advantageously treated. For example, if these steps are carried out in a suitable biological sample (possibly isolated from said patient), e. g. a serum or blood preparation, and this preparation is readministered to the patient after carrying out both the agonist and antagonist phases, a concerted effect can be achieved. The preferred sequence of the phases is to be decided.

Further examples for the diverse effects of CD1d restricted T-cells or iNKT cells, as well as methods for differentiating between responses elicited by said cells are described e. g. in Brigl et al, 2004, Moodycliffe et al, 2000, Smyth et al, 2000 and publications referred therein.

New techniques for the isolation, in vitro differentiation and manipulation of DCs offers unlimited possibilities for the pharmacological manipulation and antigen loading of these rare cells. The manipulated DCs than can be re-introduced to the patient and used for immunotherapy. Taken the short half life and the continuous generation of DC precursors the timing and regime of the therapy can be scheduled and the number of injections can be designed. Thus, the wide spread pharmacological effects of PPARg can be targeted to DCs and the unwanted adverse effects can be avoided.

The method for isolation and in vitro expansion and differentiation of human DCs from the human bone marrow and splenic mouse DCs was described in 1992 (Inaba et al., 1992, Caux et al, 1992). The technology of developing sufficient numbers of therapeutically relevant DC from human blood monocytes was developed by Antonio Lanzavecchia and Gerold Schuler (Sallusto et al., 1994, Romani et al., 1996).

The clinical application of adoptive DC-based vaccines requires the optimization of ex vivo procedures in preclinical studies. The major steps of the preparation of a personalized DC-based vaccine are as follows: i) Isolation of sufficient numbers of DC precursors, ii) Successful ex vivo generation of functionally potent immature DC, iii) Freezing and storage of ex vivo generated immature DC without affecting their functional activity, iv) Efficient recovery of frozen immature DC for further activation, v) Efficient activation of autologous DC with migratory and T cell activating capacity to generate a cellular vaccine for, optionally repeated, vaccination of the patient.

It is preferred if functionally relevant human DC vaccines satisfy the following requirements: phenotypic characteristics, which determine certain functional activity; differentiation state, which allows the migration to draining lymph nodes, where T cell activation can occur; effector functions (expression of cell surface molecules, chemokine receptors, production of cytokines), which ensure the proper activation, polarization and expansion of both helper and cytotoxic T lymphocytes.

Our preclinical studies revealed, that monocyte-derived immature DC generated in vitro by the method described herein results in fully potent DC. This was verified by the detailed phenotypic analysis of immature and mature DC as compared to monocytes.

**Autologous cell therapy and vaccination against cancers**

The discovery of DC and the description of their functional complexity and flexibility together with the availability of recombinant cytokines led to a rapid expansion of novel tumor-specific therapies. The clinical trials designed for the utility of DC as natural adjuvants of tumor associated antigens (TAA) were designed by considering the recent results of DC biology and tumor-specific immunity. Data accumulated in the past 5-6 years revealed that DC-based immunotherapies are feasible, since :

DC of various origin can be isolated in sufficient quantities from human peripheral blood, reintroduction of TAA-loaded DC is well tolerated, tumor-specific immunological responses could be detected in certain but not in all patients.

In personalized DC-based tumor-specific vaccination, besides the steps described above, preferably tumor associated antigens are selected and isolated in a sufficient amounts either from self tumor tissue or as a recombinant protein or synthetic peptide for loading immature DC. Thereafter loading with tumor antigens and the appropriate activation of autologous DC is carried out.

According to the present invention the DCs can be manipulated as described herein.

**a. Monitoring the effect of DC vaccination**

Novel techniques developed for analyzing cellular immune responses should be aimed at both quantifying the antigen-specific T lymphocyte responses and identifying the phenotype and function of effector cells (Yee and Greenberg, 2002). The use of labeled MHC-peptide multimers enables to estimate the number of T cells, which carry TAA-specific TCR. Quantitative real time polymerase chain reaction (Q-RT-PCR) can be used for the detection of clone-specific regions of the TCR. These approaches, however, do not provide information on the functional activity of the antigen-specific T lymphocytes, which is of particular interest in the case of tumor infiltrating cells. Functional assays detect antigen-specific effector T lymphocytes on the basis of their cytokine production (ELISPOT), RNA-ase protection assay or Q-RT-PCR for the quantitation of cytokine gene expression. These assays, however, usually do not detect naive, anergic or functionally inactive T cells. Intracellular cytokine detection by flow cytometry enables the quantitation of effector T cells in combination with phenotype identification.

**b. Dendritic cell-basedin vivo vaccination**

The adaptation of DC-based vaccination strategies for in vivo application can be a promising future strategy. DC could be targeted in vivo by using delivery systems described e. g. by (Biragyn et al., 1999; Syrengelas et al, 1996; Fushimi et al., 2000). An in situ LC vaccine, used for the induction of tumor-specific protective immunity in mice, was also described (Kumamoto et al., 2002). In situloading of DC with tumor antigens in combination with immunostimulatory sequences (ISS) and Flt-3 ligand resulted in long-term anti-tumor immunity in mice (Merad et al., 2002). Gene transfer in DC with an oral bacterial vector resulted in protective immunity against murine fibrosarcoma (Paglia et al., 1998). Gene gun technology was successfully used for in vivo transfection of a tumor antigen skin-derived LC, which migrated to the draining lymph nodes (Rea et al., 2001). The ISS content of DNA vaccines served as a potent activator of immature DC and supported their mobilization and differentiation.

**Exemplary method for DC-based immunotherapy**

In vivo or ex vivo manipulation of DCs allows the DCs to re-induce or maintain immunological tolerance, or to stimulate cellular immune responses or modulate inflammatory or anti-inflammatory mechanisms. In these settings:

1) Autologous DC precursors are separated from the peripheral blood of patients by magnetic or other isolation techniques.

2) Ex vivo differentiation of DC precursors to immature and/or to mature DCs in the presence of cytokine cocktails.

3) Ex vivo DC maturation can be targeted by various drugs, in this case by RSG to generate the desired phenotype and function.

4) After appropriate modification and/or activation the modulated autologous DCs can be re-introduced to the patient.

The advantage of this approach would be to avoid the drug's effect on other target cells. RSG is known to act on various cell types and to exert various biological functions.

Depending on the functional activity of manipulated DCs autologous cell therapy may have a tolerizing effect or may support antigen-specific inflammatory responses.

The skilled person will understand that a suitable combination of the therapeutic possibilities of the invention can be used for the targeted manipulation of the immune response. Moreover, the combination of DC-based and conventional anti-tumor therapies would open up further possibilities. For example, radiation and chemotherapy of primary or residual tumors result in the apoptotic and/or necrotic death of tumor cells and thus may increase the local concentration of TAA and provide danger stimuli for resident DC. IFN-alpha is widely used for the treatment of various cancers (Pfeffer et al.1998), it acts directly on tumor cells and indirectly on the host's immune response by enhancing both humoral and cellular immune responses and supporting long term anti-tumor activity (Ferrantini and Belardelli, 2000)

**Experimental procedures**

**Ligands**

Rosiglitazone, Wy14643 and T0901317 were obtained from Alexis Biochemicals, oxidized LDL from Intracel. GW501516, GW347845X and GW9662 were provided by T. M. Willson (GlaxoSmithKline, Research Triangle Park, NC) and alpha-GalCer was obtained from Kirin Brewery Ltd. (Gunma, Japan).

**DC generation and maturation**

Highly enriched monocytes (98% CD14+) were obtained from buffy coats of healthy donors (provided by the local blood center) by Ficoll gradient centrifugation and immunomagnetic cell separation using anti-CD14-conjugated microbeads (VarioMACS; Miltenyi Biotec). Human monocyte-derived IDCs were prepared as described previously (Sallusto and Lanzavecchia, 1994) with minor modification. In brief, monocytes were resuspended into six-well culture dishes at a density of 1. 5x 106 cells/ml and cultured in RPMI 1640 supplemented with 10% FBS (Invitrogen), containing 800 U/ml GM-CSF (Leucomax) and 500 U/ml IL-4 (Peprotech). Cells were cultured for 5 or 6 days and the IL-4 and GM-CSF addition was repeated at day 2 to obtain a population of immature DCs. In some experiments FBS was replaced with human AB serum (Sigma). To obtain MDCs, IDCs were treated with a mixture of cytokines : 10 ng/ml TNF-alpha (Peprotech), 10 ng/ml IL-lbeta (Peprotech), 1000 U/ml IL-6 (Peprotech), 1 microg/ml PGE (Sigma) and 800 U/ml GM-CSF for 24 hr (Jonuleit et al., 1997).

Peripheral blood myeloid DCs were magnetically isolated with the CD1c (BDCA-1) Dendritic Cell Isolation Kit (Miltenyi Biotec) from monocyte depleted PBMC. Blood myeloid-DCs (2x105 cells/well) were cultured for 2 days in RPMI 1640 supplemented with 10% FBS (Invitrogen) in the absence of exogenous cytokines.

Ligands or vehicle control (50% DMSO/ethanol) were added to the cell culture starting from the first day.

Immunohistochemistry For immune localization studies, serial cryosections of tissue samples of human tonsils were used after 4% paraformaldehyde (pH 7.2) fixation (Tontonoz et al., 1998). Endogenous peroxidase was blocked with 0.1 M periodic acid for 10 min. Monoclonal anti-PPARg antibody (E8 ; Santa-Cruz) and polyclonal rabbit antibody specific for the S-100 protein (Novocastra) were used. Following incubation with primary and biotinylated secondary antibodies, ABC peroxidase was used according to the manufacturer's instructions. Peroxidase activity was visualized by the Vector VIP (Vector)-substrate. For doublestaining sequential immuno labelling was performed using the Envision system (DAKO) according to the manufacturer's instruction. Briefly, monoclonal antibody to PPARg was visualized with Nickel enhanced-DAB followed by immunostaining of S-100 protein using the VIP substrate. Sections were counterstained with 0.2% methylgreen.

**Quantitation of IFMg Production by ELISA**

Supernatants of PBMC cultures were stored at-80°C until they were analyzed for the presence of IFNg. Cytokine levels were measured by IFNg a Biosource ELISA kit (Biosource) according to the manufacturer's instructions.

**Western blot analysis.**

Protein extracts from cells were prepared by lysing the cells in buffer A (Tris-HCl pH 7.5, 1 mM EDTA, 15 mM beta-mercaptoethanol, 0.1% Triton X 100, 0. 5mM PMSF). 20 microg protein was separated by electrophoresis in 10% polyacrylamide gel and then transferred to PVDF membrane (Bio-Rad Laboratories). Membranes were probed with anti-PPARg (H-100) antibody (Santa Cruz Biotechnology) then the membranes were stripped and reprobed with anti-GAPDH antibody (Sigma) according to the manufacturer's recommendations. Immunoblots were developed by using a chemiluminescent detection (ECL) system (Amersham).

**FACS analysis**

Cell staining was performed using alpba-GalCer-loaded APC-labeled CD1d-tetramer (kindly provided by A. Herbelin) and FITC-, Cyc-or PE-conjugated mAbs. Labeled antibodies for flow cytometry included anti-CD161-Cyc, CD8-PE, CD25-PE, CD1a-PE, CD1d-PE, CD86-PE, CD83-PE, CD206-PE, CD3-FITC, CD36-FITC, CD80-FITC, CD209-FITC, HLA-DR-FITC and isotype-matched controls (BD PharMingen), anti-Vbeta11-PE and anti-Valpha24-FITC (Immunotech). The cells were assessed for fluorescence intensity using EPICS Elite flow cytometer (Beckman Coulter) or with FACS Calibur cytometer (Beckton Dickinson). Data analysis was performed using WinMDI software (Joseph Trotter, The Scripps Research Institute, La Jolla, CA).

**Endocytosis**

FITC-dextran (Sigma) was used to measure mannose receptor-mediated endocytosis. Cells were incubated with 1 mg/ml FITC-dextran for 1 h at 37°C (control at 0 °C) and the uptake of FITC-dextran was determined by flow cytometry. Phagocytosis was measured by the cellular uptake of Latex beads (Sigma, carboxylate modified, mean diameter 1 microm) : cells were incubated with latex beads for 4 or 24 h at 37°C (control at 0°C), washed twice and the uptake was quantified by FACS.

**Mixed leukocyte reaction**

MDCs were collected, extensively washed and used as stimulator cells for allogeneic PBMC (2x10⁵ cells/well). Stimulator cells were added in graded doses to the T cells in 96-well flat-bottom tissue culture plates. Cell proliferation was measured on day 5 by a 16 h pulse with [³H]-thymidine (Amersham, 1 microCi/well).

**Expansion of iNKT cells**

IDCs were treated with or without the inflammatory mixture as described previously and with 100 ng/ml alpha-GalCer for 24 h to obtain alpha-GalCer-loaded MDC or IDC, respectively. alpha-GalCer pulsed IDCs or MDCs (2x10⁵ cells/ml) were cocultured with 2x10⁶ monocyte depleted autologous PBMC for 5 days in 24 well plates. The expansion of iNKT cells was monitored by quantifying Valpha24+ Vbeta1 1+ and Valpha24+CD161+cells by FACS analysis. The specificity of CD1d-restricted NKT cells was also verified by alpha-GalCer loaded APC-labelled CD1d tetramer staining.

**Microarray experiment**

Total RNA was isolated using Trizol Reagent (Invitrogen) and further purified by using the RNeasy kit (Quiagen). cRNA was generated from 5 microg of total RNA by using the Superscript Choice kit (Invitrogen) and the High Yield RNA transcription labeling kit (Enzo Diagnostics). Fragmented cRNA was hybridized to Affymetrix (Santa Clara, CA) arrays (HU95A) according to Affymetrix standard protocols. Preliminary data analysis was performed by the Microarray Core Facility at University of California at Irvine. Further analysis was performed using GeneSpring 6.0 (Silicon Genetics, Redwood City, CA). These analyses provided a signal for each specific transcript that was subsequently normalized by comparing to the median signal (arbitrary value of 1.0) obtained from the whole array.

**Real-time RT-PCR**

Total RNA was isolated with TRIZOL reagent (Invitrogen) and RNA was treated with RNase free DNase (Promega) before reverse transcription. Reverse transcription was performed at 42°C for 30 min from 100 ng of total RNA using Superscript II reverse transcriptase (Invitrogen) and gene specific reverse primer. Quantitative PCR analysis was performed using real-time PCR (ABI PRISM 7900 sequence detector, Applied Biosystems) performing 40 cycles of 95°C for 12 sec and 60°C for 1 min using Taqman assays. All PCR reactions were done in triplicates with one control reaction containing no RT enzyme to test for potential DNA contamination. The comparative Ct method was used to quantify various transcripts to the control endogenous gene 36B4.36B4 Ct values did not vary between cell types or treatments. To assess the efficiency of the amplification we always included with 10-fold dilutions of synthetic gene-specific amplicon (triplicate) on every plate. The following primers and probes were used:

FABP4 (NM001442):

(294+) GGATGGAAAATCAACCACCA,

(375-) GGAAGTGACGCCTTTCATGA,

PAM-ATTCCACCACCAGTTTATCATCCTCTCGTT,

CD1d (NM001766):

(957+) GAGGCCCCACTTTGGGTAA,

(1025-) CACTGTTTCCCTCGTCCACTT,

FAM-TGGCCATTCAAGTGCTCAACCAGG-TAMRA,

CD1a (NM_001763):

(1357-) ACCTGTCCTGTCGGGTGAA,

(1435+) CCCACGGAACTGTGATGCT,

FAM-CAGTCTAGAGGGCCAGGACATCGTCCT-TAMRA,

PPARg (NM005037) :

(1313+) GATGACAGCGACTTGGCAA,

(1397-) CTTCAATGGGCTTCACATTCA,

FAM-CAAACCTGGGCGGTCTCCACTGAG-TAMRA,

PPARa (NM005036):

(459+) CATTACGGAGTCCACGCGT,

(527-) ACCAGCTTGAGTCGAATCGTT,

FAM-AGGCTGTAAGGGCTTCTTTCGGCG,

PPARd (NM006238):

(595+) AGCATCCTCACCGGCAAAG,

(660-) CCACAATGTCTCGATGTCGTG,

FAM-CAGCCACACGGCGCCCTTTG-TAMRA

36B4 (NM001002) :

(193+) AGATGCAGCAGATCCGCAT,

(322-) ATATGAGGCAGCAGTTTCTCCAG,

FAM-AGGCTGTGGTGCTGATGGGCAAGAA-TAMRA

Further methods for isolating, manipulating APC-s and precursors, as well as examples for detecting or studying manipulated APCs are described e. g. in W004015056.

**Results**

**PPARg is an immediate early gene during monocyte-derived DC differentiation**

In order to provide a baseline for our studies we first examined the expression pattern and dynamics of the different PPAR isoforms in monocytes and monocyte-derived DCs. Monocytes were cultured in the presence of GM-CSF and IL-4 for 5 days to generate immature DCs (IDCs). First we assessed the mRNA levels of PPAR receptors in monocytes and IDCs using real-time quantitative RT-PCR. By using this technique transcript levels of the various receptors could be directly compared. PPARg was barely detectable in freshly isolated monocytes but it was significantly up-regulated during DC differentiation (Figure 1A). The transcript level of PPARd was also increased during this differentiation process (Figure 1A). The mRNA level of PPARa was relatively low in monocytes and only slightly increased in IDCs (Figure 1A). Based on these results we concluded that in monocyte-derived DCs the dominant isotypes of PPARs are PPARg and PPARd. It should be noted that PPARg was induced to a larger extent than PPARd during the course of differentiation. Interestingly, we found a very rapid increase of PPARg mRNA already after 1 hour in culture reaching its highest level between 2-4 hours and then decreasing slightly by 24 hours (Figure 1 B). We also assessed the protein expression of PPARg by immunoblot. As shown in Figure 1D, immunoblotting of whole cell extracts revealed that after 4 hours PPARg protein was present and after 12h in culture we detected a substantial increase of PPARg protein in line with the increased mRNA expression levels.

**PPARg is functional and active in DCs and can be further activated by exogenous ligands**

To verify whether PPARg is already active or can be activated during DC development, cells were treated with specific agonists of PPARg and mRNA expression of known target genes of this receptor were measured by RT-PCR. Our microarray data indicated that the adipocyte specific fatty acid binding protein (FABP4 or aP2), a target gene of PPARg (Tontonoz et al., 1995) was a sensitive marker of PPARg activity (data not shown). Therefore we used it as an indicator of endogenous activation of PPARg, both as an endpoint of assessing the presence of endogenous ligands, and as the measure of exogenous ligand activation. First we looked at the expression of PPARg and FABP4 during the course of DC differentiation. As shown on Figure 1B PPARg is acutely up-regulated within 1 hour and this is followed by the induction of FABP4 after 4 hours suggesting that the receptor is activated by endogenous ligands (Figure 1C). These data indicate that some endogenous ligand/activator of PPARg may be present or are generated during this differentiation process. To address this issue we used a specific antagonist of PPARg (GW9662) for inhibiting target gene expression such as FABP4. GW9662 was able to prevent the up-regulation of FABP4 (Figure 1E) in both untreated and ligand-treated cells. Next we looked at the effect of a synthetic thiazolidinedione, Rosiglitazone (RSG) on PPARg regulated gene expression. The compound was added to the culture upon the initiation of DC differentiation (zero point of time). The transcript level of FABP4 was consistently and significantly up-regulated as a result of ligand treatment (Figure IE). Other target genes such as LXRalpha or PGAR (Chawla et al., 2001b ; Yoon et al., 2000) were also up-regulated after treatment with the PPARg specific agonist (data not shown). It was also noted that FABP4 levels got induced to a higher level in human AB serum than in FBS (fetal bovine serum) (Figure 1E) suggesting that human AB serum contains and/or induces endogenous ligands/activators. This more pronounced increase could also be blocked effectively by the PPARg antagonist (Figure 1E). We also monitored the degree of PPARg responsiveness during the course of DC differentiation. Differentiation was initiated and the specific ligand was added at points of time 0, day 1,2, 3 or 4 and FABP4 expression levels were determined (Figure 1F). The highest level of response could be achieved if the cells were exposed to the ligand in the first 24 hours of differentiation. This coincides with the highest level of PPARg expression (Figure 1B). These results established that PPARg is promptly expressed at high levels in differentiating DCs, it is transcriptionally active, and most likely it is activated by endogenous ligand (s). The highest level of receptor expression and ligand responsiveness occurs in the first 24 hours of differentiation.

To assess the consequences of PPARg activation for DC differentiation and function we carried out an extensive analysis of differentiation markers, gene expression profiles and functional studies (antigen uptake, T cell activation, cytokine production).

**PPARg agonist modulate the expression of DC specific membrane proteins**

We measured the cell surface expression of monocyte and DC specific membrane proteins by flow cytometry. In agreement with a previous publication (Nencioni et al., 2002) addition of the PPARg activator induced changes of the pattern of cell surface molecules: CD86 and HLA-DR expression were increased whilst CD80 and CD1a were decreased on IDCs generated in the presence of PPARg specific ligand (data not shown). It is important to note, that ligand treatment was most efficient if added at the beginning of the differentiation process (Figure 1F). As a consequence of ligand treatment IDCs remained CD14 negative and the expression of DC-SIGN (CD209) was unchanged or in some cases marginally decreased (data not shown). These data indicate that RSG treatment does not inhibit but modifies DC differentiation and as a consequence the expression of some DC markers is changed. Next we studied the effect of PPARg activation on key functions of DC.

**RSG treated immature DCs display enhanced endocytotic activity**

Endocytosis is a hallmark of immature DCs, therefore we were interested to test if activation of PPARg had any influence on this activity. The capacity of RSG treated immature DCs to take up antigens was measured by two methods: 1) Internalization of FITC-dextran, which is mainly taken up by mannose receptor mediated endocytosis, and 2) Engulfment of latex beads for detection of phagocytosis. RSG did not influence FITC-dextran dependent endocytosis of DC but enhanced the uptake of latex beads (Figure 2A). It should be emphasized that monocytes also possess high capacity of latex bead uptake and RSG treated cells retained this activity. In immature DCs a number of cell surface receptors are involved in antigen uptake. One of them is CD36, which may act as a receptor for apoptotic cell uptake and has been postulated to mediate cross priming of cytotoxic T cells by human DCs (Albert et al., 1998). It is also a known target of PPARg) (Nagy et al.; 1998). We examined the cell surface expression of CD36 during DC development and detected higher expression of CD36 on PPARg activator treated IDCs than on control cells (Figure 2A). The detailed analysis of CD1a and CD36 expression in correlation with latex bead uptake revealed that CD1a negative cells had a higher capacity of engulfing latex beads than CD1a positive cells (Figure 2B). We observed that in some individuals RSG treated DCs could be divided into high and low expressors of CD36. However, CD36'°W cells had similar capacity as highly positive cells to uptake latex beads (data not shown) suggesting that CD36 expression may not account for increased uptake of latex beads. CD206 (mannose receptor) levels were induced in IDCs and remained unaltered by PPARg activator treatment, which further underscores the notion that PPARg activation does not inhibit DC differentiation. Mannose receptor, which is involved in FITC-dextran uptake is up-regulated during DC differentiation (Sallusto et al., 1995). A recent paper suggested that PPARg ligand may promote mannose receptor gene expression in murine macrophages (Coste et al., 2003), in human monocyte-derived DC we could not detect up-regulation of this protein upon RSG treatment. (Figure 2A).

In line with previous results (Sallusto et al., 1995) mannose receptor, which is involved in FITC-dextran uptake, was induced in IDCs (Figure 2A) and remained unaltered by PPARg activator treatment, which further underscores the notion that PPARg activation does not inhibit DC differentiation. A recent paper suggested that PPARg ligand may promote mannose receptor gene expression in murine macrophages (Coste et al., 2003), but in human monocyte-derived DC we could not detect up-regulation of this protein upon RSG treatment.

**RSG treated MDCs have an unaltered T lymphocyte stimulatory capacity**

An important aspect of DC function is the ability of DCs to activate T cells. We first determined the effect of RSG on the expression of surface molecules on mature DC. As shown in Figure 3A MDCs become CD83 positive and up-regulate the expression of HLA-DR and the co-stimulatory molecules CD80 and CD86. Upon maturation of DCs in the presence of a PPARg specific activator CD80 was slightly down-regulated but the expression of the other cell surface molecules tested on MDC did not changed (Figure 3A). Next we assessed the stimulatory capacity of MDCs to elicit proliferative responses of alloreactive T lymphocytes. We found that the stimulatory capacity of RSG treated mature DC for T cells was comparable to that of control MDC (Figure 3B). To further explore the potential effects of PPARg activation of DC on lymphocyte activation and cytokine production we measured IFNg production in Mixed Leukocyte Reaction (MLR) by ELISA (Figure 3C) and also performed two-color flow cytometric analysis of T cells to assess the expression of CD25 a lymphocyte activation marker (Figure 3D). We observed that PPARg ligand (2.5 uM RSG) treatment of the differentiating DCs did not impair IFNg production and activation of allogeneic T lymphocytes. These results indicate that PPARg ligand treatment does not impede the T cell stimulatory capacity of the DCs and does not interfere with the maturation of DCs.

**Activation of PPARg coordinately modulates the expression of CD1s during DC differentiation**

After assessing the effect of PPARg activation on known genes implicated in DC differentiation and function we embarked on a more comprehensive analysis of gene expression using global gene expression profiling. An important family of genes showing differential regulation was the CD1 family. Intriguingly, the CD1 proteins have been implicated in lipid antigen presentation (Porcelli, 1995).

CD1a is highly up-regulated during monocyte-derived DC differentiation using FBS containing cell-culture medium. We and others observed that addition of RSG at the initiation of DC differentiation resulted in marked inhibition of CD1a expression (Nencioni et al., 2002) and data not shown. Since the most pronounced effect of PPARg activators on DC was the selective down regulation of CD la and this change correlated with a significantly increased endocytic capacity, we analyzed the ligand's effect on the expression of the entire CD1 gene family. We determined the transcript level of CD1 family members during DC differentiation using Affymetrix microarray analysis (HU95A). The CD1 locus encodes a family of conserved transmembrane proteins structurally related to MHC class I proteins (Calabi et al., 1989). We found that freshly isolated monocytes devoid of expression not only CD1 but also the rest of the group I CD1s (CD1b, CD1c and CD1e). The transcript levels of these genes were strongly increased on immature DC and as a result of ligand treatment the mRNA levels of these genes were much lower (Figure 4A). Unexpectedly, CD1d (group II CD1) showed a completely different expression pattern. Monocytes were CD1d positive but DC expressed very low levels of CD1d mRNA (Figure 4B). RSG treated cells showed a retained CD1d expression. The typical expression pattern of CD1a and CD1d was confirmed by RT-PCR (Figure 4C and D) in six independent samples. To assess the receptor specificity of this regulation we used a panel of synthetic receptor-specific agonists to see if activation of PPARa (Wy16643), PPARd (GW501516), and LXRs (T0901317), which are also expressed in differentiating DCs, produce a similar effect on CD1d expression. We also used other structurally distinct synthetic and natural PPARg agonists/activators (GW7845, oxidized LDL) alone or in combination with the PPARg antagonist (GW9662) to prove the receptor selectivity of the effect. We compared the expression of a bone fide target gene FABP4 to that of CD1d. As shown on Figure 5A induction of FABP4 is specifically up-regulated by PPARg agonists and much less potently by a PPARa activator. The regulation of CD1d showed a similar, but distinct pattern. Besides PPARg activators (RSG, GW7845), PPARd (GW501516) and PPARa (Wy14, 643) activators could also turn on gene expression though to a lesser extent (Figure 5B). The PPARg specific induction of FABP4 and CD1d was inhibited by the specific antagonist (Figure 5C and D). It was reported that PPARg activation induces LXRalpha in macrophages (Chawla et al., 2001b) and we confirmed this result in DC (data not shown) so it is possible that CD1d got activated indirectly via the activation of LXRalpha. Our data show that this is unlikely to be the case because the LXR activator (T0901317) failed to activate CD1d (Figure 5B). We previously described that the naturally occurring, pathogenic lipoprotein oxLDL contains PPARg agonists (Nagy et al., 1998) and we observed that oxLDL induced FABP4 and CD1d expression in DCs (Figure 5C and D). It is noteworthy that oxLDL induced CD1d levels to a much higher level than indicated by its potential to activate PPARg (compare Figure 5C and D). This suggests that oxLDL may utilize both a PPARg-dependent and independent pathway for turning on CD1d.

We compared the kinetics of the induction of FABP4 and CD1d during the course of DC differentiation. We observed that after 12 hours FABP4 was highly induced upon ligand treatment (Fig 5E). The freshly isolated monocytes express a significant amount of CD1d and it was downregulated during the beginning of DC differentiation regardless of the PPARg agonist treatment. After 12 hours we measured very low expression of CD1d, but after 5 days we detected a net induction of CD1d in PPARg activated cells (Figure 5F). These data suggest that PPARg activators not simply attenuate the decreased expression of CD1d but instead, probably indirectly, induce CD1d expression in DCs.

**RSG treated DC express more CD1d and have an improved capacity to induce iNKT cell expansion**

The induced CD1d and the reduced CD1a protein expression was detected on the cell surface of PPARg agonist (RSG) treated IDCs and MDCs as shown in Figure 6A and B in agreement with the mRNA expression. The finding that RSG treated monocyte-derived DCs express more CD1d mRNA and protein then untreated cells focused our attention on the physiological consequence of increased CD1d expression on DC. CD1d mediated glycolipid presentation is indispensable for the activation and expansion of iNKT cells (Brossay et al., 1998). We reasoned that increased CD1d protein levels should translate into increased activation of iNKT cell. We assessed the relative potency of DCs to induce iNKT cell proliferation in autologous MLR cultures. DCs were pulsed with alpha-GalCer in vitro for 24 hours with or without an inflammatory cocktail to obtain alpha-GalCer loaded MDCs or IDCs respectively and used as stimulatory cells for monocyte-depleted autologous PBMC in a 5-day culture. We determined the percent of iNKT cells using two-color flow cytometry for the detection of the cells harboring Valpha24+ Vbeta11 + invariant T cell receptors. We observed that, alpha-GalCer was necessary for the potent induction of Valpha24+ Vbeta11 + cells expansion. Without this glycolipid we obtained less than 1% iNKT cells after 5 days of coculture (data not shown). However, we detected a remarkable expansion of iNKT cells when the cells were loaded with alpha-GalCer and this was further enhanced in presence of RSG treated IDC (Figure 6C). We repeated this experiment several times and obtained inductions ranging between 1.5-10. 25 fold depending on the donor. Interestingly, using MDCs as stimulator we consistently observed a more moderate effect of the PPARg ligand treatment on iNKT expansion ranging between 1.1-4 fold. In order to characterize the population of iNKT cells induced by RSG treated DCs we also determined the ratio of Valpha24+ CD161+ cells. We detected a very similar ratio of double positive cells if compared alphaGalCer loaded control and RSG treated DCs (Figure 6C). Furthermore the specificity and the contribution of the various NKT subsets to the induced cell population was proved by alpha-GalCer loaded CD1d tetramer staining in combination with anti-CD4 or anti-CD8 antibodies. Human NKT cells can be divided into three subsets DN, CD4+ or CD8+ subpopulations (Gumperz et al., 2002; Takahashi et al., 2002). We have determined the distribution of these three subsets in the induced iNKT cell pool and found that the ratio of the three subsets was unchanged even though the number of CD1d tetramer positive cells increased if RSG treated IDCs were used as stimulators.

**PPARg can be detected in cultured myeloid blood derived DC and in inter-digitating DCs of tonsils.**

In this study we examined in detail the expression profile of PPARg in monocyte-derived DCs. To extend this study into other in vivo relevant cells we also investigated the expression of PPARg in blood DCs. Dendritic cells can be purified directly from human peripheral blood, which represent at least three different subsets (Dzionek et al., 2000). We isolated CD11c+ myeloid DCs from monocyte-depleted PBMC with a yield of 2-6x10⁶ DCs/500 ml blood. These cells were CD14-CD11c+ and expressed CD1d (data not shown). First we determined the transcript level of PPARg in blood DCs using RT-PCR. We found that freshly isolated myeloid DC did not express PPARg but after two days in culture PPARg was upregulated. The expression level was 20-30% of monocyte-derived DC (Figure 7A). Next we examined the effects of PPARg ligand (2.5 IJM RSG) on the expression of FABP4 and CD1d in blood DCs. FABP4 was slightly upregulated in PPARg ligand treated cells (Figure 7B). We also assessed CD1d expression and observed that cultured DC express less CD1d than freshly isolated cells but the ligand treated cells showed an increased expression of CD1d (Figure 7C). The expression pattern of CD1d was very similar to that of monocyte-derived DCs although the induction was lower probably due to the lower level of PPARg expression.

Our results indicate that freshly isolated monocytes and blood derived DCs lack PPARg, but in vitro differentiated and cultured cells express this gene at high levels. It is reasonable to assume that in vivo transmigration and/or the local environment contributes to PPARg induction. Factors involved in this induction remain to be identified. In order to test this scenario we attempted to detect PPARg protein in human lymphoid tissue (tonsil) by immunohistochemitry. As shown in Figure 7D nuclear expression of PPARg protein could be detected close to the surface epithelium of the tonsil, a typical site for the s to reside (see below). A few PPARg-positive cells were also found to be surrounded by lymphoid cells of the perifollicular zones of the tonsil (Figure 7D insert) indicating DC-T cell interactions. We also stained a similar section with anti-S-100 antibody, a marker of activated interdigitating DCs (Laman et al., 2003; Shimizu et al., 2002). Both staining decorated the non-epithelial s with similar distribution patterns (compare Figure 7D to 7E). Some cells, which co-expressed the nuclear PPARg (Figure 7E Inset, arrow) and the cytoplasmic S-100 protein (arrowhead) could also be found in the lymphoid compartment. By contrast, no staining was seen in the nuclei of lymphocytes. As a positive control for PPARg immunostaining we detected the PPARg protein in human brown fat cells obtained from hibernoma Figure 7F. Negative control for the staining specificity, sections were incubated with preimmune mouse serum instead of the specific polyclonal antibody did not show immunostaining (data not shown). These results support that at least a subset of S-100+ interdigitating DCs express PPARg in vivo.

INDUSTRIAL APPLICABILITY

These findings are entirely consistent with the new mechanism described herein and warrant further investigation into the details of this pathway and its utility in the therapy of autoimmune diseases.

**References**:

Albert, M. L., Pearce, S. F., Francisco, L. M., Sauter, B., Roy, P., Silverstein, R. L., and Bhardwaj, N. (1998). Immature dendritic cells phagocytose apoptotic cells via alpbavbeta5 and CD36, and cross-present antigens to cytotoxic T lymphocytes. J Exp Med 188, 1359-1368.

Augstein, P., Dunger, A., Heinke, P., Wachlin, G., Berg, S., Hehmke, B., and Salzsieder, E. (2003). Prevention of autoimmune diabetes in NOD mice by troglitazone is associated with modulation of ICAM-1 expression on pancreatic islet cells and IFN-gamma expression in splenic T cells. Biochem Biophys Res Commun 304, 378-384.

Banchereau, J., and Steinman, R. M. (1998). Dendritic cells and the control of immunity. Nature 392, 245-252.

Biragyn, A., Tani, K., Grimm, M. C., Weeks, S., and Kwak, L. W. (1999). Genetic fusion of chemokines to a self tumor antigen induces protective, T-cell dependent antitumor immunity. Nat Biotechnol 17, 253-258.

Brigle, M. and Brenner, M. B., (2004) CD1 : Antigen presentation and T cell function. Annu. Rev. Immunol. 22: 817-890.

Brossay, L., Chioda, M., Burdin, N., Koezuka, Y., Casorati, G., Dellabona, P., and Kronenberg, M. (1998). CDld-mediated recognition of an alpha-galactosylceramide by natural killer T cells is highly conserved through mammalian evolution. J Exp Med 188, 1521-1528.

Calabi, F., Jarvis, J. M., Martin, L., and Milstein, C. (1989). Two classes of CD1 genes. Eur J Immunol 19, 285-292.

Carnaud, C., Gombert, J., Donnars, O., Garchon, H., and Herbelin, A. (2001). Protection against diabetes and improved NK/NKT cell performance in NOD. NK1.1 mice congenic at the NK complex. J Immunol 166, 2404-2411.

Caux C., Dezutter-Dambuyant C, Schmitt D, Banchereau J.: GM-CSF and TNF-alpha cooperate in the generation of dendritic Langerhans cells. Nature 360,258-261, (1992)

Chawla, A., Barak, Y., Nagy, L., Liao, D., Tontonoz, P., and Evans, R. M. (2001a). PPAR-gamma dependent and independent effects on macrophage-gene expression in lipid metabolism and inflammation. Nat Med 7, 48-52.

Chawla, A., Boisvert, W. A., Lee, C. H., Laffitte, B. A., Barak, Y., Joseph, S. B., Liao, D., Nagy, L., Edwards, P. A., Curtiss, L. K., et al. (2001b). A PPAR gamma-LXR-ABCA1 pathway in macrophages is involved in cholesterol efflux and atherogenesis. Mol Cell 7, 161-171.

Chen, H., and Paul, W. E. (1997). Cultured NK1.1+ CD4+ T cells produce large amounts of IL-4 and IFN-gamma upon activation by anti-CD3 or CD1. J Immunol 159, 2240-2249.

Coste, A., Dubourdeau, M., Linas, M. D., Cassaing, S., Lepert, J. C., Balard, P., Chalmeton, S., Bernad, J., Orfila, C., Seguela, J. P., and Pipy, B. (2003). PPARgamma promotes mannose receptor gene expression in murine macrophages and contributes to the induction of this receptor by IL-13. Immunity 19, 329-339.

Delerive, P., Fruchart, J. C., and Staels, B. (2001). Peroxisome proliferator-activated receptors in inflammation control. J Endocrinol 169, 453-459.

Desreumaux, P., Dubuquoy, L., Nutten, S., Peuchmaur, M., Englaro, W., Schoonjans, K., Derijard, B., Desvergne, B., Wahli, W., Chambon, P., et al. (2001). Attenuation of colon inflammation through activators of the retinoid X receptor (RXR)/peroxisome proliferator-activated receptor gamma (PPARgamma) heterodimer. A basis for new therapeutic strategies. J Exp Med 193, 827-838.

Dzionek, A., Fuchs, A., Schmidt, P., Cremer, S., Zysk, M., Miltenyi, S., Buck, D. W., and Schmitz, J. (2000). BDCA-2, BDCA-3, and BDCA-4 : three markers for distinct subsets of dendritic cells in human peripheral blood. J Immunol 165, 6037-6046.

Exley, M., Garcia, J., Balk, S. P. and Porcelly, S., (1997). Requirements for CD1d recognition by human invariant Valpha24+ CD4-CD8-T cells. J. Exp. Med. 186 : 109-20.

Exley, M., Garcia, J., Wilson, S. B., Spada, F., Gerdes, D., Tahir, S. M., Patton, K. T., Blumberg, R. S., Porcelli, S., Chott, A., and Balk, S. P. (2000). CD1d structure and regulation on human thymocytes, peripheral blood T cells, B cells and monocytes. Immunology700, 37-47.

Faveeuw, C., Fougeray, S., Angeli, V., Fontaine, J., Chinetti, G., Gosset, P., Delerive, P., Maliszewski, C., Capron, M., Staels, B., et al. (2000). Peroxisome proliferator-activated receptor gamma activators inhibit interleukin-12 production in murine dendritic cells. FEBS Lett 486, 261-266.

Fushimi, T., Kojima, A., Moore, M. A., and Crystal, R. G. (2000). Macrophage inflammatory protein 3 alpha transgene attracts dendritic cells to established murine tumors and suppresses tumor growth. J Clin Invest 105,1383-1393.

Gosset, P., Charbonnier, A.S., Delerive, P., Fontaine, J., Staels, B., Pestel, J., Tonnel, A. B., and Trottein, F. (2001). Peroxisome proliferator-activated receptor gamma activators affect the maturation of human monocyte-derived dendritic cells. Eur J Immunol 31, 2857-2865.

Gumperz, J. E., Miyake, S., Yamamura, T., and Brenner, M. B. (2002). Functionally distinct subsets of CD1d-restricted natural killer T cells revealed by CD1d tetramer staining. J Exp Med 195, 625-636.

Hammond, K. J., and Kronenberg, M. (2003). Natural killer T cells: natural or unnatural regulators of autoimmunity? Curr Opin Immunol 15, 683-689.

Hsi, L. C., Wilson, L., Nixon, J., and Eling, T. E. (2001). 15-lipoxygenase-1 metabolites down-regulate peroxisome proliferator-activated receptor gamma via the MAPK signaling pathway. J Biol Chem 276, 34545-34552.

Huang, J. T., Welch, J. S., Ricote, M., Binder, C. J., Willson, T. M., Kelly, C., Witztum, J. L., Funk, C. D., Conrad, D., and Glass, C. K. (1999). Interleukin-4-dependent production of PPAR-gamma ligands in macrophages by 12/15-lipoxygenase. Nature 400, 378-382.

Inaba K., Inaba M., Romani N., Aya H., Deguchi M., Ikehara S., Muramatsu S., Steinman R. M.: Generation of large numbers of dendritic cells from mouse bone marrow cultures supplemented with granulocyte/macrophage colony-stimulating factor. J. Exp. Med. 176,1693-1702, (1992)

Jiang et al. Nature 391, 82 (1998). <BR> <BR> <BR> <P>[363] Jonuleit, H., Kuhn, U., Muller, G., Steinbrink, K., Paragnik, L., Schmitt, E., Knop, J., and Enk, A. H. (1997). Pro-inflammatory cytokines and prostaglandins induce maturation of potent immunostimulatory dendritic cells under fetal calf serum-free conditions. Eur J Immunol 27, 3135-3142.

Kasinrerk, W., Baumruker, T., Majdic, O., Knapp, W., and Stockinger, H. (1993). CD1 molecule expression on human monocytes induced by granulocyte-macrophage colony-stimulating factor. J Immunol 150, 579-584.

Kawano, T., Cui, J., Koezuka, Y., Toura, I., Kaneko, Y., Motoki, K., Ueno, H., Nakagawa, R., Sato, H., Kondo, E., et al. (1997). CD1d-restricted and TCR-mediated activation of valpha14 NKT cells by glycosylceramides. Science 278, 1626-1629.

Kronenberg, M., and Gapin, L. (2002). The unconventional lifestyle of NKT cells. Nat Rev Immunol 2, 557-568.

Kukreja, A., Costi, G., Marker, J., Zhang, C. H., Sinha, S., Sun, Z., and Maclaren, N. (2002). NKT cell defects in NOD mice suggest therapeutic opportunities. J Autoimmun 19, 117-128.

Kumamoto, T., Huang, E. K., Paek, H. J., Morita, A., Matsue, H., Valentini, R. F., and Takashima, A. (2002). Induction of tumor-specific protective immunity by in situ langerhans cell vaccine. Nat Biotechnol 20, 64-69.

Laman, J. D., Leenen, P. J., Annels, N. E., Hogendoorn, P. C., and Maarten Egeler, R. (2003). Langerhans-cell histiocytosis'insight into DC biology'. Trends Immunol 24, 190-196.

Liu, Y. J. (2001). Dendritic cell subsets and lineages, and their functions in innate and adaptive immunity. Cell 106, 259-262.

Merad, M., Sugie, T., Engleman, E. G., and Fong, L. (2002). In vivo manipulation of dendritic cells to induce therapeutic immunity. Blood 99, 1676-1682.

Moody, D. B., and Porcelli, S. A. (2003). Intracellular pathways of CD1 antigen presentation. Nat Rev Immuno13, 11-22.

Moody, D. B., Besra, G. S., Wilson, I. A., and Porcelli, S. A. (1999). The molecular basis of CD1-mediated presentation of lipid antigens. Immunol Rev 172, 285-296.

Moodycliffe, A. M., Ngiem, D., Clydesdale, G. and Ullrich, S. E., (2000) Immune suppression and skin cancer development. Nature Immunology 1 (6) 521-525.

Moser, M. (2003). Dendritic cells in immunity and tolerance - do they display opposite functions? Immunity 19, 5-8.

Motojima, Cell Structure and Function, 18, 267-277,1993

Nagy, L., Tontonoz, P., Alvarez, J. G., Chen, H., and Evans, R. M. (1998). Oxidized LDL regulates macrophage gene expression through ligand activation of PPARgamma. Cell 93, 229-240.

Nencioni, A., Grunebach, F., Zobywlaski, A., Denzlinger, C., Brugger, W., and Brossart, P. (2002). Dendritic cell immunogenicity is regulated by peroxisome proliferator-activated receptor gamma. J Immunol 169, 1228-1235.

Olweus J., BitMansour A., Wamke R., Thompson P. A., Carballido J., Picker L. J., Lund-Johansen F. (1997) Dendritic cell ontogeny: A human dendritic cell lineage of myeloid origin. Proc. Nat. Acad. Sci. U. S. A. 94, 12551-12556.

Paglia, P., Medina, E., Arioli, I., Guzman, C. A., and Colombo, M. P. (1998). Gene transfer in dendritic cells, induced by oral DNA vaccination with Salmonella typhimurium, results in protective immunity against a murine fibrosarcoma. Blood 92, 3172-3176.

Perrin-Cocon, L., Coutant, F., Agaugue, S., Deforges, S., Andre, P., and Lotteau, V. (2001). Oxidized low-density lipoprotein promotes mature dendritic cell transition from differentiating monocyte. J Immunol 167, 3785-3791.

Porcelli, S. A. (1995). The CD1 family: a third lineage of antigen-presenting molecules. Adv Immuno159, 1-98.

Porcelli, S., Morita, C. T., and Brenner, M. B. (1992). CD1b restricts the response of human CD4-8-T lymphocytes to a microbial antigen. Nature 360,593-597.

Randolph, G. J., Beaulieu, S., Lebecque, S., Steinman, R. M., and Muller, W. A. (1998). Differentiation of monocytes into dendritic cells in a model of transendothelial trafficking. Science 282, 480-483.

Randolph, G. J., Inaba, K., Robbiani, D. F., Steinman, R. M., and Muller, W. A. (1999). Differentiation of phagocytic monocytes into lymph node dendritic cells in vivo. Immunity 11, 753-761.

Rea, D., Johnson, M. E., Havenga, M. J., Melief, C. J., and Offringa, R. (2001). Strategies for improved antigen delivery into dendritic cells. Trends Mol Med 7, 91-94.

Repa, J. J., Turley, S. D., Lobaccaro, J. A., Medina, J., Li, L., Lustig, K., Shan, B., Heyman, R. A., Dietschy, J. M., and Mangelsdorf, D. J. (2000). Regulation of absorption and ABC1-mediated efflux of cholesterol by RXR heterodimers. Science 289, 1524-1529.

Ricote et al., Nature, 391. 79 (1998).

Romani N., Reider D., Heuer M., Ebner S., Kampgen E., Eib1 B., Niederwieser D., Schuler G.: Generation of mature dendritic cells from human blood. An improved method with special regard to clinical applicability. J Immunol Methods. 196, 137-151, (1996).

Sallusto F. and Lanzavecchia A.: Efficient presentation of soluble antigen by cultured human dendritic cells is maintained by granulocyte/macrophage colony-stimulating factor plus interleukin 4 and downregulated by tumor necrosis factor alpha. J. Exp. Med. 179,1109-18, (1994).

Sallusto, F., Cella, M., Danieli, C., and Lanzavecchia, A. (1995). Dendritic cells use macropinocytosis and the mannose receptor to concentrate macromolecules in the major histocompatibility complex class II compartment: downregulation by cytokines and bacterial products. J Exp Med 182, 389-400.

Sharif, S., Arreaza, G. A., Zucker, P., Mi, Q. S., Sondhi, J., Naidenko, O. V., Kronenberg, M., Koezuka, Y., Delovitch, T. L., Gombert, J. M., et al. (2001). Activation of natural killer T cells by alpha-galactosylceramide treatment prevents the onset and recurrence of autoimmune Type 1 diabetes. Nat Med 7,1057-1062.

Shi, F. D., Flodstrom, M., Balasa, B., Kim, S. H., Van Gunst, K., Strominger, J. L., Wilson, S. B., and Sarvetnick, N. (2001). Germ line deletion of the CD1 locus exacerbates diabetes in the NOD mouse. Proc Natl Acad Sci U S A 98, 6777-6782.

Shimizu, S., Yoshinouchi, T., Ohtsuki, Y., Fujita, J., Sugiura, Y., Banno, S., Yamadori, I., Eimoto, T., and Ueda, R. (2002). The appearance of S-100 protein-positive dendritic cells and the distribution of lymphocyte subsets in idiopathic nonspecific interstitial pneumonia. Respir Med 96, 770-776.

Sieling, P. A., Jullien, D., Dahlem, M., Tedder, T. F., Rea, T. H., Modlin, R. L., and Porcelli, S. A. (1999). CD1 expression by dendritic cells in human leprosy lesions : correlation with effective host immunity. J Immunol 162, 1851-1858.

Smyth, M. I., and Godfrey, D. I., (2000) NKT cells and tumor immunity-a double edged sword. Nature Immunology 1 (6) 459-460.

Spanbroek, R., Hildner, M., Kohler, A., Muller, A., Zintl, F., Kuhn, H., Radmark, O., Samuelsson, B., and Habenicht, A. J. (2001). IL-4 determines eicosanoid formation in dendritic cells by down-regulation of 5-lipoxygenase and up-regulation of 15-lipoxygenase 1 expression. Proc Natl Acad Sci U S A 98, 5152-5157.

Spiegelman, Diabetes, 47,507 (1998),

Summers, K. L., Hock, B. D., McKenzie, J. L., and Hart, D. N. (2001). Phenotypic characterization of five dendritic cell subsets in human tonsils. Am J Pathol 159, 285-295.

Syrengelas, A. D., Chen, T. T., and Levy, R (1996). DNA immunization induces protective immunity against B-cell lymphoma. Nat Med 2, 1038-1041.

] Takahashi, T., Chiba, S., Nieda, M., Azuma, T., Ishihara, S., Shibata, Y., Juji, T., and Hirai, H. (2002). Cutting edge: analysis of human V alpha 24+CD8+ NK T cells activated by alpha-galactosylceramide-pulsed monocyte-derived dendritic cells. J Immunol 168, 3140-3144.

Tontonoz et al. Cell, 93,241, (1998).

Tontonoz, P., Hu, E., and Spiegelman, B. M. (1995). Regulation of adipocyte gene expression and differentiation by peroxisome proliferator activated receptor gamma. Curr Opin Genet Dev 5, 571-576.

Tontonoz, P., Nagy, L., Alvarez, J. G., Thomazy, V. A., and Evans, R. M. (1998). PPARgamma promotes monocyte/macrophage differentiation and uptake of oxidized LDL. Cell 93, 241-252.

Urban, B. C., Willcox, N., and Roberts, D. J. (2001). A role for CD36 in the regulation of dendritic cell function. Proc Natl Acad Sci U S A 98, 8750-8755.

Vincent, M. S., Gumperz, J. E., and Brenner, M. B. (2003). Understanding the function of CD1-restricted T cells. Nat Immunol 4, 517-523.

Vincent, M. S., Leslie, D. S., Gumperz, J. E., Xiong, X., Grant, E. P., and Brenner, M. B. (2002). CD1-dependent dendritic cell instruction. Nat Immunol 3, 1163-1168.

Wang, B., Geng, Y. B., and Wang, C. R. (2001). CD1-restricted NK T cells protect nonobese diabetic mice from developing diabetes. J Exp Med 194, 313-320.

Willson et al., J. Med. Chem., 43, 527 (2000)

Willson, T. M., Lambert, M. H., and Kliewer, S. A. (2001). Peroxisome proliferator-activated receptor gamma and metabolic disease. Annu Rev Biochem 70, 341-367.

Wilson, S. B. and Delovitch, T. L. (2003) Janus-like role of regulatory iNKT cells in autoimmune disease and tumour immunity. Nature Reviews, Immunology 3 211-222.

Yee C. and Greenberg P. D.: Modulating T-cell immunity to tumors: New strategies for monitoring T-cell responses. Nature. Rev. Cancer. 2,409-419, 2002

Yoon, J. C., Chickering, T. W., Rosen, E. D., Dussault, B., Qin, Y., Soukas, A., Friedman, J. M., Holmes, W. E., and Spiegelman, B. M. (2000). Peroxisome proliferator-activated receptor gamma target gene encoding a novel angiopoietin-related protein associated with adipose differentiation. Mol Cell Biol 20, 5343-5349.

## Claims

1. A manipulated professional antigen presenting cell (APC) wherein said manipulated professional APC is a dendritic cell (DC) and wherein
in said cell the expression or activity of an endogenous peroxisome proliferator activated receptor gamma (PPARg) is increased,
said cell having increased expression of a CD1 type II molecule, and
having decreased expression of at least one of the following CD1 type I molecules: CD1a, CD1b and CD1c,
relative to a control non-manipulated DC
wherein the activation of allogeneic T-lymphocytes and IFNg production is not impaired if tested in Mixed Leukocyte Reaction (MLR).

2. The manipulated APC of claim 1 wherein
- the PPARg is modulated by ligand induced activation or by increasing the expression of said PPARg,
- the CD 1 type II molecule is a CD 1d molecule, and
- the CD 1 type I molecule is a CD 1a molecule.

3. The manipulated APC of claim 2 which is selected from a DC of myeloid origin, a monocyte derived DC or an interdigitating DC of lymphoid tissue, e.g. of tonsils.

4. The manipulated DC of claim 3
which is a mature DC (MDC), or
which is an immature DC (IDC).

5. The manipulated professional APC of any of claims 1 to 4 said APC being capable of activating CD 1d restricted iNKT cells.

6. The manipulated immature or mature APC of any of claims 1 to 5 for use in autologous cell therapy.

7. The manipulated APC of any of claims 1 to 5 for use in the treatment of microbial infections.

8. The manipulated immature or mature APC of any of claims 1 to 5
for use for inducing tolerance in the treatment of tissue specific or systemic autoimmune diseases, in allergies, hypersensitivity reactions or post-transplant conditions, or
for use in the treatment of autoimmune diseases, e.g. type 1 diabetes, multiple scleroses, autoimmune encephalomyelitis, anterior chamber-associated immune deviation (ACAID), lupus erithematosus, autoimmune hepatitis, inflammation conditions.

9. The manipulated mature APC of any of claims 1 to 5 for use in the treatment of neoplastic diseases, e.g. skin cancer, hematological tumours, colorectal carcinoma, or a tumorous condition treatable by IFN-g.

10. Use of a PPARg agonist in the preparation of a manipulated APC of any of claims 1 to 9.

11. A kit for manipulating a DC, *in vitro,* comprising at least the following:
- a PPARg agonist compound,
- means for isolating DC precursor cells, e.g. blood monocyte cells or monocyte derived cells or a precursor thereof,
- one or more reagent for detecting altered expression of a CD 1 type II molecule.

## Patentansprüche

1. Manipulierte professionelle antigenpräsentierende Zelle (APC), wobei die manipulierte professionelle APC eine dendritische Zelle (DC) ist und wobei
in der Zelle die Expression oder Aktivität eines endogenen Peroxixomproliferator-aktivierten Rezeptors-gamma (PPARg) erhöht ist,
wobei die Zelle eine erhöhte Expression eines CD1-Typ-II-Moleküls aufweist und
eine verringerte Expression mindestens eines der folgenden CD1-Typ-I-Moleküle aufweist: CD1a, CD1b und CD1 c,
in Bezug auf eine nicht manipulierte Kontroll-DZ,
wobei die Aktivierung von allogenen T-Zellen und die IFN-g-Produktion bei einem Test in einer gemischten Leukozytenreaktion (MLR) nicht beeinträchtigt sind.

2. Manipulierte APC nach Anspruch 1, wobei
- der PPARg durch ligandeninduzierte Aktivierung oder durch Erhöhen der Expression des PPARg moduliert wird,
- das CD1-Typ-II-Molekül ein CD1d-Molekül ist und
- das CD1-Typ-I-Molekül ein CD1a-Molekül ist.

3. Manipulierte APC nach Anspruch 2, die aus einer DC mit Myeloidursprung, einer Monozyten-abgeleiteten DC oder einer interdigitierenden DC von lymphatischem Gewebe, z. B. von den Mandeln, ausgewählt ist.

4. Manipulierte DC nach Anspruch 3,
bei der es sich um eine reife DC (MDZ) handelt oder
bei der es sich um eine unreife DC (IDZ) handelt.

5. Manipulierte professionelle APC nach einem der Ansprüche 1 bis 4, wobei die APC CD1d-restringierte iNKT-Zellen aktivieren können.

6. Manipulierte unreife oder reife APC nach einem der Ansprüche 1 bis 5 zur Verwendung in der autologen Zelltherapie.

7. Manipulierte APCnach einem der Ansprüche 1 bis 5 zur Verwendung bei der Behandlung von mikrobiellen Infektionen.

8. Manipulierte unreife oder reife APC nach einem der Ansprüche 1 bis 5 zur Verwendung zum Induzieren von Toleranz bei der Behandlung gewebespezifischer oder systemischer Autoimmunerkrankungen, Überempfindlichkeitsreaktionen oder Zuständen nach einer Transplantation oder
zur Verwendung bei der Behandlung von Autoimmunerkrankungen, z. B. Typ-I-Diabetes, multiple Sklerosen, autoimmune Enzephalomyelitis, Abweichung der Immunantwort der Vorderkammer (ACAID), Lupus erythematodes, autoimmune Hepatitis, Entzündungszustände.

9. Manipulierte reife APZ nach einem der Ansprüche 1 bis 5 zur Verwendung bei der Behandlung von Tumorleiden, z. B. Hautkrebs, hämatologische Tumoren, kolorektales Karzinom oder ein durch IFN-g behandelbarer Tumorzustand.

10. Verwendung eines PPARg-Agonisten bei der Herstellung einer manipulierten APC nach einem der Ansprüche 1 bis 9.

11. Kit zum Manipulieren einer DC *in vitro,* das mindestens folgendes umfasst:
- eine PPARg-Agonistenverbindung,
- Mittel zum Isolieren von DC-Vorläuferzellen, z. B. Blut-Monozytenzellen oder Monozyten-abgeleitete Zellen oder ein Vorläufer davon,
- ein oder mehrere Reagenzien zum Nachweisen einer veränderten Expression eines CD1-Typ-II-Moleküls.

## Revendications

1. Cellule présentatrice d'antigène professionnelle manipulée (APC), dans laquelle ladite APC professionnelle manipulée est une cellule dendritique (DC), et dans laquelle
dans ladite cellule, l'expression ou l'activité d'un récepteur gamma activé par les proliférateurs des peroxysomes endogène (PPARg) est augmentée,
ladite cellule ayant une expression accrue d'une molécule CD1 de type II, et ayant une expression réduite d'au moins l'une des molécules CD1 de type I suivantes : CD1a, CD1b et CD1c,
par rapport à une DC témoin non manipulée
dans laquelle l'activation de la production des lymphocytes T allogéniques et de l'IFNg n'est pas affectée si testée dans une réaction leucocytaire mixte (MLR).

2. APC manipulée selon la revendication 1, dans laquelle
- le PPARg est modulé par une activation induite par un ligand ou par une augmentation de l'expression dudit PPARg,
- la molécule CD1 de type II est une molécule CD1d, et
- la molécule CD1 de type I est une molécule CD1 a.

3. APC manipulée selon la revendication 2, qui est choisie parmi une DC d'origine myéloïde, une DC dérivée de monocyte ou une DC interdigitée de tissu lymphoïde, par exemple des amygdales.

4. DC manipulée selon la revendication 3
qui est une DC mature (MDC), ou
qui est une DC immature (IDC).

5. APC professionnelle manipulée selon l'une quelconque des revendications 1 à 4, ladite APC étant capable d'activer les cellules iNKT restreintes au CD1d.

6. APC immature ou mature manipulée selon l'une quelconque des revendications 1 à 5 destinée à une utilisation en thérapie cellulaire autologue.

7. APC manipulée selon l'une quelconque des revendications 1 à 5 destinée à une utilisation dans le traitement des infections microbiennes.

8. APC immature ou mature manipulée selon l'une quelconque des revendications 1 à 5
destinée à une utilisation pour induire une tolérance dans le traitement des maladies auto-immunes spécifiques d'un tissu ou systémiques, dans les allergies, les réactions d'hypersensibilité ou les affections post-greffe, ou
destinée à une utilisation dans le traitement des maladies auto-immunes, par exemple, le diabète de type 1, la sclérose en plaques, l'encéphalomyélite auto-immune, la déviation immune associée à la chambre antérieure (ACAID), le lupus érythémateux, l'hépatite auto-immune, les états inflammatoires.

9. APC mature manipulée selon l'une quelconque des revendications 1 à 5 destinée à une utilisation dans le traitement des maladies néoplasiques, par exemple le cancer de la peau, les tumeurs hématologiques, le carcinome colorectal, ou une affection tumorale traitable par l'IFN-g.

10. Utilisation d'un agoniste de PPARg dans la préparation d'une APC manipulée selon l'une quelconque des revendications 1 à 9.

11. Trousse pour la manipulation d'une DC, *in vitro,* comprenant au moins les éléments suivants :
- un composé agoniste de PPARg,
- un moyen pour isoler des cellules précurseurs de DC, par exemple des cellules monocytaires sanguines ou des cellules dérivées de monocytes ou un précurseur de celles-ci,
- un ou plusieurs réactifs pour la détection de l'expression modifiée d'une molécule CD1 de type II.
